# EUROPEAN PATENT APPLICATION

(11) **EP 1 757 686 A1**
(43) Date of publication of application: **28.02.2007**
(21) Application number: 04726750.5
(22) Date of filing: 09.04.2004
(51) Int. Cl.: C12N 15/09, C07K 16/28, C07K 16/46, A61P 35/00, A61P 37/02, A61P 43/00, A61K 39/395

(54) **CELL DEATH INDUCER**

(71) Applicant: CHUGAI SEIYAKU KABUSHIKI KAISHA, Tokyo, 115-8543 (JP); Ozaki, Shuji, Tokushima-shi, Tokushima 770-0804 (JP); Abe, Masahiro, Tokushima-shi, Tokushima 770-0033 (JP)
(72) Inventor: OZAKI, Shuji, Tokushima-shi, Tokushima 7700804 (JP); ABE, Masahiro, Tokushima-shi, Tokushima 7700033 (JP); TSUCHIYA, Masayuki, CHUGAI SEIYAKU KABUSHIKI K., Gotenba-shi, Shizuoka 4128513 (JP); KIMURA, Naoki, CHUGAI SEIYAKU KABUSHIKI KAISHA, Gotenba-shi, Shizuoka 4128513 3 (JP); KAWAI, Shigeto, CHUGAI SEIYAKU KABUSHIKI KAISHA, Gotenba-shi, Shizuoka 4128513 (JP)
(74) Representative: Vossius & Partner
(86) International application number: PCT/JP2004/005152
(87) International publication number: WO 2005/100560

(57) **Abstract**

To identify antigens of the 2D7 antibody, the present inventors cloned the 2D7 antigen. As a result, the 2D7 antibody was found to recognize HLA class IA. In addition, the present inventors examined whether the 2D7 antibody has cell death-inducing activity. Nuclei fragmentation was observed when the 2D7 antibody was cross-linked with another antibody, indicating that cell-death was induced. Further, diabodies of the 2D7 antibody were found to have very strong cell death-inducing activities, even without the addition of another antibody. These results indicate that minibodies of an HLA-recognizing antibody can be used as cell death-inducing agents.

## Description

### Technical Field

The present invention relates to minibodies that recognize HLA.

### Background Art

The HLA class I antigen is formed by a heterodimer of a 45-KD α chain comprising three domains (α1, α2, α3), and a 12-KD β2 microglobulin. The main role of the HLA molecule is to present CD8⁺T cells with antigenic peptides formed from about eight to ten amino acids and produced inside cells. As such, it plays a very important role in the immune response and immune tolerance induced by this peptide presentation.

Cell growth-suppressing and cell death-inducing effects have been observed in lymphocytes upon HLA class IA antigen and antibody ligation, suggesting that HLA molecules may also be signal transduction molecules.

More specifically, for example, there are reports showing cell growth suppression of activated lymphocytes by the B9.12.1 antibody against the α1 domain of human HLA class IA, the W6/32 antibody against the α2 domain, and the TP25.99 and A1.4 antibodies against the α3 domain (Non-patent Documents 1, 2). Furthermore, two types of antibodies, MoAb90 and YTH862, against the human HLA class IA α1 domain have been reported to induce apoptosis in activated lymphocytes (Non-patent Documents 2, 3, 4). Apoptosis induced by these two antibodies has been shown to be a caspase-mediated reaction (Non-patent Document 4), and therefore, HLA class IA antigens expressed in lymphocytes are also speculated to be involved in apoptosis signal transduction.

Furthermore, the 5H7 antibody against the α3 domain of human HLA class IA (Non-patent Document 5), and the RE2 antibody against the α2 domain of mouse HLA class IA (Non-patent Document 6) have been also reported to induce cell death in activated lymphocytes and the like. However, in contrast with the aforementioned apoptosis-inducing antibodies MoAb90 and YTH862, it has been shown that none of the cell deaths induced by these antibodies are caspase-mediated. Accordingly, cell deaths due to 5H7 and RE2 are predicted to be of a type completely different from conventionally known apoptosis mechanisms.

As described above, there are numerous reports of the cell growth-suppressing actions and cell death-inducing actions of anti-HLA antibodies. However, the antibodies used herein are all in the molecular forms of IgG antibodies, F(ab')2, or Fab, and to date there have been no reports that cell death-inducing activity is enhanced by reducing the molecular weight of antibodies, as in F(ab')2 and Fab.

Prior art literature relating to the present invention is shown below:
[Non-patent Document 1] Fayen et al., Int. Immunol. 10: 1347-1358(1998)
[Non-patent Document 2] Genestier et al., Blood 90: 3629-3639 (1997)
[Non-patent Document 3] Genestier et al., Blood 90: 726-735 (1997)
[Non-patent Document 4] Genestier et al., J. Biol. Chem. 273: 5060-5066 (1998)
[Non-patent Document 5] Woodle et al., J. Immunol. 158: 2156-2164 (1997)
[Non-patent Document 6] Matsuoka et al., J. Exp. Med. 181: 2007-2015 (1995)
[Non-patent Document 7] Goto, et al. Blood 84: 1922 (1994)

### Disclosure of the Invention

The primary purpose of this invention is to provide minibodies of antibodies that recognize HLA class IA. A further objective of this invention is to provide novel therapeutic agents for tumors or autoimmune diseases that utilize these minibodies.

The present inventors obtained 2D7 antibodies recognizing HLA class IA. Then, it was examined whether the 2D7 antibodies have cell death-inducing activity. More specifically, Jurkat cells were cultured in the presence or absence of 2D7, with anti-mouse IgG antibody also added. Cell nuclei were stained 48 hours later with Hoechst 33258, and then checked for cell nuclei fragmentation, which is characteristic of dead cells. As a result, hardly any cell death-inducing activity was observed in Jurkat cells with 2D7 antibody alone; however, nuclei fragmentation was observed when the antibody was further cross-linked with anti-mouse IgG antibody, showing that cell death was induced.

As described, because cross-linking with an anti-mouse IgG antibody is necessary for 2D7 antibody to induce cell death, it is difficult to clinically apply the 2D7 antibody to tumors or autoimmune diseases. Therefore, the present inventors examined the effect of reducing the molecular weight of the 2D7 antibody on cell death induction. More specifically, genes encoding the variable regions of the 2D7 antibody were cloned from hybridomas. The 2D7 antibody was then made into diabodies using genetic engineering techniques and effects on cell death-inducing activity were examined. Surprisingly, the 2D7 antibody converted to diabodies showed strong cell death-inducing activity within a very short time and at low doses, even without cross-linking with an anti-mouse IgG antibody. Furthermore, the diabody hardly acted on normal peripheral blood-derived lymphocytes and adherent cells, and specifically induced cell death in various myeloma cells, T cell leukemia cell lines, and activated lymphocytes. The above-mentioned results show that the minibodies of antibodies recognizing HLA can be utilized as cell death-inducing agents.

More specifically, the present invention provides the following [1] to [14]:
[1] a minibody, which comprises a heavy chain variable region comprising CDRs 1, 2, and 3 which consist of the amino acid sequences of SEQ ID NOs: 13, 14, and 15;
[2] a minibody which is functionally equivalent to the minibody of [1], and which comprises heavy chain CDRs consisting of amino acid sequences with one or more amino acid substitutions, deletions, insertions, and/or additions in the amino acid sequences of the heavy chain CDRs of the minibody of [1];
[3] a minibody, which comprises a light chain variable region comprising CDRs 1, 2, and 3 which consist of the amino acid sequences of SEQ ID NOs: 16, 17, and 18;
[4] a minibody which is functionally equivalent to the minibody of [3], and which comprises light chain CDRs consisting of amino acid sequences with one or more amino acid substitutions, deletions, insertions, and/or additions in the amino acid sequences of the light chain CDRs of the minibody of [3];
[5] a minibody, which comprises a heavy chain variable region comprising CDRs 1, 2, and 3 which consist of the amino acid sequences of SEQ ID NOs: 13, 14, and 15, and a light chain variable region comprising CDRs 1, 2, and 3 which consist of the amino acid sequences of SEQ ID NOs: 16, 17, and 18;
[6] a minibody which is functionally equivalent to the minibody of [5], and which comprises CDRs consisting of amino acid sequences with one or more amino acid substitutions, deletions, insertions, and/or additions in the amino acid sequences of the CDRs of the minibody of [5];
[7] the minibody of any one of [1] to [6], which is a diabody;
[8] a cell death-inducing agent comprising the minibody of any one of [1] to [7] as an active ingredient;
[9] the cell death-inducing agent of [8], which induces cell death of a B-cell or T-cell;
[10] the cell death-inducing agent of [9], wherein the B-cell or T-cell is an activated B-cell or an activated T-cell;
[11] a cell growth-suppressing agent comprising the minibody of any one of [1] to [7] as an active ingredient;
[12] an anti-tumor agent comprising the minibody of any one of [1] to [7] as an active ingredient;
[13] the anti-tumor agent of [12], wherein the tumor is blood tumor; and
[14] an agent for treating an autoimmune disease, wherein the agent comprises the minibody of any one of [1] to [7] as an active ingredient.

The present invention provides minibodies that recognize HLA. The minibodies of this invention are useful since their activity is elevated. Herein activity refers to a biological action that is caused by binding an antibody to an antigen. Specific examples include cell death-inducing actions, apoptosis-inducing actions, cell growth-suppressing actions, cell differentiation-suppressing actions, cell division-suppressing actions, cell growth-inducing actions, cell differentiation-inducing actions, cell division-inducing actions, and cell cycle-regulating actions. Cell death-inducing actions and cell growth-suppressing actions are preferred.

The cells that become the target of the above-mentioned actions, such as cell death-inducing actions and cell growth-suppressing actions, are not particularly limited, though blood cells and non-adherent cells are preferred. Specific examples of blood cells include lymphocytes (B cells, T cells), neutrophils, eosinophils, basophils, monocytes (preferably activated peripheral blood mononuclear cells (PBMC)), and myeloma cells, while lymphocytes (B cells, T cells), and myeloma cells are preferred, and T cells or B cells (particularly activated B cells or activated T cells) are most preferable. Non-adherent cells refer to cells that, when cultured, grow in a suspended state without adhering to the surface of culturing vessels of glass, plastic or the like. On the other hand, adherent cells refer to cells that, when cultured, adhere to the surface of culturing vessels of glass, plastic or the like.

In the present invention, administration of the minibodies that recognize HLA can treat or prevent diseases such as tumors, including blood tumors (hematopoietic tumors) (specific examples include leukemia, myelodysplastic syndrome, malignant lymphoma, chronic myelogenic leukemia, plasmacytic disorders (myeloma, multiple myeloma, macroglobulinemia), and myeloproliferative diseases (polycythemia vera, essential thrombocythemia, idiopathic myelofibrosis)), and autoimmune diseases (specific examples include rheumatism, autoimmune hepatitis, autoimmune thyroiditis, autoimmune bullosis, autoimmune adrenocortical disease, autoimmune hemolytic anemia, autoimmune thrombycytopenic purpura, autoimmune atrophic gastritis, autoimmune neutropenia, autoimmune orchitis, autoimmune encephalomyelitis, autoimmune receptor disease, autoimmune infertility, Crohn's disease, systemic lupus erythematosus, multiple sclerosis, Basedow's disease, juvenile diabetes, Addison's disease, myasthenia gravis, lens-induced uveitis, psoriasis, and Behchet's disease).

In the present invention, HLA refers to human leukocyte antigen. HLA molecules are categorized into class I and class II. Known examples of class I are HLA-A, B, C, E, F, G, H, J, and such; and known examples of class II are HLA-DR, DQ, DP, and such. The antigens recognized by the antibodies of this invention are not particularly limited, so long as they are HLA molecules, preferably molecules classified as class I, and more preferably HLA-A.

In the present invention, a minibody comprises an antibody fragment that lacks a portion of a whole antibody (for example, whole IgG). The minibodies of the present invention are not particularly limited so long as they can bind an antigen. There are no particular limitations on the antibody fragments of the present invention, so long as they are portions of a whole antibody, and preferably contain a heavy chain variable region (VH) or a light chain variable region (VL). More preferably, the antibody fragments contain both a heavy chain variable region (VH) and a light chain variable region (VL). Specific examples of the antibody fragments include Fab, Fab', F(ab')2, Fv, and scFv (single chain Fv), but are preferably scFv (Huston, J. S. et al., Proc. Natl. Acad. Sci. U.S.A. (1988) 85, 5879-5883; Plickthun "The Pharmacology of Monoclonal Antibodies" Vol. 113, Resenburg and Moore Ed., Springer Verlag, New York, pp. 269-315, (1994)). Such antibody fragments can be prepared by treating an antibody with an enzyme, such as papain or pepsin for example, to generate antibody fragments, or by constructing genes that encode these antibody fragments, introducing them into expression vectors, and then expressing them in appropriate host cells (see, for example, Co, M. S. et al., 1994, J. Immunol. 152, 2968-2976; Better, M. and Horwitz, A. H., 1989, Methods Enzymol. 178, 476-496; Pluckthun, A. and Skerra, A., 1989, Methods Enzymol. 178, 497-515; Lamoyi, E., 1986, Methods Enzymol. 121, 652-663; Rousseaux, J. et al., 1986, Methods Enzymol. 121, 663-669; Bird, R. E. and Walker, B. W., 1991, Trends Biotechnol. 9,132-137).

The minibodies of this invention preferably have smaller molecular weights than a whole antibody; however, they may form multimers, including dimers, trimers, and tetramers, and the molecular weights may become greater than that of the whole antibody.

A preferred minibody of this invention is an antibody comprising two or more antibody VHs and two or more antibody VLs, in which each of these variable regions is linked directly or indirectly via linkers and such. Such linkages may be covalent bonds or non-covalent bonds, or may be both. An even more preferable minibody is an antibody comprising two or more VH-VL pairs formed by non-covalent bonding between VH and VL. In this case, the distance between one VH-VL pair and another VH-VL pair is preferably shorter in a minibody than in a whole antibody.

A particularly preferable minibody of this invention is a diabody. A diabody is a dimer formed by binding two fragments, in which a variable region is linked to another variable region via a linker and such (for example, scFv) (hereinafter referred to as diabody-constituting fragments), and usually comprises two VLs and two VHs (P. Holliger et al., Proc. Natl. Acad. Sci. USA, 90, 6444-6448 (1993); EP404097; WO93/11161; Johnson et al., Method in Enzymology, 203, 88-98, (1991); Holliger et al., Protein Engineering, 9, 299-305, (1996); Perisic et al., Structure, 2, 1217-1226, (1994); John et al., Protein Engineering, 12(7), 597-604, (1999); Holliger et al., Proc. Natl. Acad. Sci. USA., 90, 6444-6448, (1993); Atwell et al., Mol. Immunol. 33, 1301-1312, (1996)). The bonds between the diabody-constituting fragments may be non-covalent or covalent bonds, but are preferably non-covalent bonds.

Alternatively, diabody-constituting fragments may be bound by a linker and such to form a single chain diabody (sc diabody). In such cases, linking the diabody-constituting fragments using a long linker of about 20 amino acids allows non-covalent bond formation between diabody-constituting fragments on the same chain, thus forming a dimer.

Diabody-constituting fragments include those with a linked VL-VH, linked VL-VL, and linked VH-VH, and are preferably those with a linked VH-VL. In the diabody-constituting fragments, the linker used to link a variable region to a variable region is not particularly limited, but is preferably a linker short enough to prevent non-covalent bonding between variable regions in the same fragment. The length of such a linker can be appropriately determined by those skilled in the art, and is ordinarily 2 to 14 amino acids, preferably 3 to 9 amino acids, and most preferably 4 to 6 amino acids. In this case, linkers between a VL and VH encoded on the same fragment are short, and thus a VL and VH on the same strand do not form a non-covalent bond nor a single-chain V region fragment; rather, the fragment forms a dimer with another fragment via non-covalent bonding. Furthermore, according to the same principle as in diabody construction, three or more diabody-constituting fragments may be bound to form multimeric antibodies, such as trimers and tetramers.

The present invention's minibodies recognizing HLA-A include minibodies that contain heavy chain variable regions comprising CDRs 1, 2, and 3 which consist of the amino acid sequences of SEQ ID NOs: 13, 14, and 15, respectively. Also included are minibodies that are functionally equivalent to the above minibodies and which comprise heavy chain CDRs consisting of amino acid sequences with one or more amino acid substitutions, deletions, insertions, and/or additions in the heavy chain CDR amino acid sequences of the above minibodies.

In addition, the minibodies of the present invention include minibodies that contain light chain variable regions comprising CDRs 1, 2, and 3 which consist of the amino acid sequences of SEQ ID NOs: 16, 17, and 18, respectively. Also included are minibodies that are functionally equivalent to the above minibodies and which comprise light chain CDRs consisting of amino acid sequences with one or more amino acid substitutions, deletions, insertions, and/or additions in the light chain CDR amino acid sequences of the above minibodies.

Preferred examples of the minibodies of the present invention include minibodies that contain heavy chain variable regions comprising CDRs 1, 2, and 3 which consist of the amino acid sequences of SEQ ID NOs: 13, 14, and 15, respectively, and light chain variable regions comprising CDRs 1, 2, and 3 which consist of the amino acid sequences of SEQ ID NOs: 16, 17, 18.

Furthermore, preferred examples of the minibodies include minibodies that are functionally equivalent to the above-described minibodies and which comprise CDRs consisting of amino acid sequences with one or more amino acid substitutions, deletions, insertions, and/or additions in the CDR amino acid sequences of the above-described minibodies.

A particularly preferred minibody of the present invention include a diabody that comprises the following amino acid sequences: AspTyrPheIleHis (SEQ ID NO: 13) as heavy chain CDR1, TrpIlePheProGlyAspAspThrThrAspTyrAsnGluLysPheArgGly(SEQ ID NO: 14) as heavy chain CDR2, SerAspAspPheAspTyr (SEQ ID NO: 15) as heavy chain CDR3, SerAlaSerSerSerValSerTyrMetHis (SEQ ID NO: 16) as light chain CDR1, SerThrSerAsnLeuAlaSer (SEQ ID NO: 17) as light chain CDR2, and GlnGlnArgThrSerTyrProProThr (SEQ ID NO: 18) as light chain CDR3.

Herein, "functionally equivalent" means that the minibody of interest has an activity equivalent to that of a diabody of interest (for example, HLA-A binding activity, and cell death-inducing activity).

The number of mutated amino acids is not particularly limited, but may usually be 30 amino acids or less, preferably 15 amino acids or less, and more preferably five amino acids or less (for example, three amino acids or less). The amino acids are preferably mutated or modified in a way that conserves the properties of the amino acid side chain. Examples of amino acid side chain properties are: hydrophobic amino acids (A, I, L, M, F, P, W, Y, and V), hydrophilic amino acids (R, D, N, C, E, Q, G, H, K, S, and T), amino acids comprising the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); aromatic ring-containing side chains (H, F, Y, and W) (amino acids are represented by one-letter codes in parentheses). Polypeptides comprising a modified amino acid sequence, in which one or more amino acid residues is deleted, added, and/or replaced with other amino acids, are known to retain their original biological activities (Mark, D. F. et al., Proc. Natl. Acad. Sci. USA 81, 5662-5666 (1984); Zoller, M. J. & Smith, M. Nucleic Acids Research 10, 6487-6500 (1982); Wang, A. et al., Science 224, 1431-1433; Dalbadie-McFarland, G. et al., Proc. Natl. Acad. Sci. USA 79, 6409-6413 (1982)). In addition, the amino acid sequences of the antibody constant regions and such are well known to those skilled in the art.

In the present invention, the HLA-recognizing minibodies specifically bind to HLA. They are not particularly limited, so long as they have a biological action. The minibodies of this invention can be prepared by methods well known to those skilled in the art. For example, as described in the Examples, the antibodies can be prepared based on the sequence of an HLA-recognizing antibody (particularly sequences of the variable regions and sequences of CDRs), using genetic engineering techniques known to those skilled in the art.

For the sequence of the HLA-recognizing antibody, particularly of the framework region (FR), a well-known antibody sequence can be used, or an anti-HLA antibody can be prepared by a method well known to those skilled in the art using HLA as the antigen, and then the sequence of this antibody can be obtained and used. Specifically, for example, this can be performed as follows: HLA protein, or a fragment thereof, is used as a sensitizing antigen to perform immunizations according to conventional immunization methods, the obtained immunocytes are fused with well-known parent cells according to conventional cell fusion methods, and monoclonal antibody-producing cells (hybridomas) are then screened by ordinary screening methods. Antigens can be prepared by known methods, such as a method using baculoviruses (WO98/46777 and such). Hybridomas can be prepared, for example, according to the method of Milstein *et al.* (Kohler, G. and Milstein, C., Methods Enzymol. (1981) 73:3-46). When the antigen has low immunogenicity, immunization can be performed using the antigen bound to immunogenic macromolecules, such as albumin. Thereafter, cDNAs of the variable region (V region) of the antibody are synthesized from the mRNAs of the hybridomas using reverse transcriptase, and the sequences of the obtained cDNAs can be determined by known methods.

Antibodies that recognize HLA are not particularly limited, so long as they bind to HLA; mouse antibodies, rat antibodies, rabbit antibodies, sheep antibodies, human antibodies, and such may be used as necessary. Alternatively, artificially modified, genetically recombinant antibodies, such as chimeric and humanized antibodies, may be used to reduce heterologous antigenicity against humans. These modified antibodies can be produced using known methods. A chimeric antibody is an antibody comprising the variable regions of the heavy and light chains of an antibody from a non-human mammal such as a mouse, and the constant regions of the heavy and light chains of a human antibody. The chimeric antibody can be produced by linking a DNA encoding the variable regions of the mouse antibody with a DNA encoding the constant regions of the human antibody, incorporating this into an expression vector, and then introducing the vector to a host.

Humanized antibodies are also referred to as "reshaped human antibodies". Such humanized antibodies are obtained by transferring the CDR of an antibody derived from a non-human mammal, for example a mouse, to the CDR of a human antibody, and general gene recombination procedures for this are also known. Specifically, a DNA sequence designed to link a murine antibody CDR to the framework region (FR) of a human antibody can be synthesized by PCR, using primers prepared from several oligonucleotides containing overlapping portions of terminal regions. The obtained DNA is linked to a DNA encoding human antibody constant regions, and this is then integrated into an expression vector, and the antibody is produced by introducing this vector into host cells (see European Patent Application EP 239400, and International Patent Application WO 96/02576). The human antibody FR to be linked via the CDR is selected so the CDR forms a favorable antigen-binding site. To form a suitable antigen-binding site in the CDR of the reshaped human antibody, amino acids in the framework region of the antibody variable region may be substituted as necessary ( Sato, K. et al., 1993, Cancer Res. 53, 851-856).

These chimeric antibodies and humanized antibodies can be chimerized, humanized, and such after their molecular weight is reduced, or their molecular weight can be reduced after they have been chimerized, humanized, or such.

Methods for obtaining human antibodies are also known. For example, human lymphocytes can be sensitized *in vitro* with a desired antigen, or with cells expressing the desired antigen, and the sensitized lymphocytes can be fused with human myeloma cells, such as U266, to obtain the desired human antibody with antigen-binding activity (Examined Published Japanese Patent Application No. (JP-B) Hei 1-59878). Further, a desired human antibody can be obtained by using a desired antigen to immunize transgenic animals that have a full repertoire of human antibody genes (see International Patent Application WO 93/12227, WO 92/03918, WO 94/02602, WO 94/25585, WO 96/34096, and WO 96/33735). Furthermore, techniques for obtaining human antibodies by panning using a human antibody library are also known. For example, variable regions of human antibodies can be expressed as single chain antibodies (scFvs) on the surface of phages using phage display methods, and phages that bind to antigens can be selected. The DNA sequences that encode the variable regions of the human antibodies binding the antigens can be determined by analyzing the genes of the selected phages. By determining the DNA sequences of the scFvs that bind to the antigens, appropriate expression vectors carrying relevant sequences can be produced to yield human antibodies. These methods are already known, and are detailed in the following publications: WO 92/01047, WO 92/20791, WO 93/06213, WO 93/11236, WO 93/19172, WO 95/01438, and WO 95/15388.

Therefore, the minibodies of the present invention can be chimerized, humanized, or such by methods well known to those skilled in the art. Such chimeric and humanized antibodies are also included in the minibodies of this invention.

The antibodies of this invention may be conjugate antibodies that are bonded to various molecules, such as polyethylene glycol (PEG), radioactive substances, and toxins. Such conjugate antibodies can be obtained by performing chemical modifications on the obtained antibodies. Methods for antibody modification are established in this field. The term "antibody" in this invention includes such conjugate antibodies.

The present invention includes DNAs that encode the antibodies of this invention. This invention also includes DNAs encoding antibodies that hybridize under stringent conditions to the aforementioned DNAs, and have antigen-binding capacity and activity. Hybridization techniques (Sambrook, J. et al., Molecular Cloning 2nd ed., 9.47-9.58, Cold Spring Harbor Lab. press, 1989) are well known to those skilled in the art, and hybridization conditions can be selected appropriately by those skilled in the art. Such hybridization conditions include, for example, conditions of low stringency. Examples of conditions of low stringency include post-hybridization washing in 0.1x SSC and 0.1% SDS at 42°C, and preferably in 0.1x SSC and 0.1% SDS at 50°C. More preferable hybridization conditions include highly stringent conditions. Highly stringent conditions include, for example, washing in 5x SSC and 0.1% SDS at 65°C. In these conditions, the higher the temperature, the higher the expectation of efficiently obtaining DNAs with a high homology. However, several factors, such as temperature and salt concentration, can influence hybridization stringency, and those skilled in the art can suitably select these factors to achieve similar stringencies.

The DNAs of this invention are used for *in vivo* and *in vitro* production of the antibodies of this invention, and for other applications, such as gene therapy. The DNAs of this invention may be in any form, so long as they encode the antibodies of this invention. More specifically, they may be cDNAs synthesized from mRNAs, genomic DNAs, chemically synthesized DNAs, or such. Furthermore, the DNAs of this invention include any nucleotide sequence based on the degeneracy of the genetic code, so long as they encode the antibodies of this invention.

The antibodies of this invention can be produced by methods well known to those skilled in the art. More specifically, a DNA of an antibody of interest is incorporated into an expression vector. In so doing, the DNA is incorporated into the expression vector and expressed under the control of an expression regulatory region such as an enhancer or promoter. Next, antibodies can be expressed by transforming host cells with this expression vector. In this regard, appropriate combinations of hosts and expression vectors can be used.

The vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs.

When using vectors to produce the antibodies of this invention, expression vectors are particularly useful. When an expression vector is expressed in *E. coli,* for example, it should have the above characteristics in order to be amplified in *E. coli.* Additionally, when *E. coli* such as JM109, DH5α, HB101, or XL1-Blue are used as the host cell, the vector preferably has a promoter, for example, a lacZ promoter (Ward et al. (1989) Nature 341:544-546; (1992) FASEB J. 6:2422-2427), araB promoter (Better et al. (1988) Science 240:1041-1043), or T7 promoter, to allow efficient expression of the desired gene in *E. coli.* Other examples of the vectors include pGEX-SX-1 (Pharmacia), "QIAexpress system" (QIAGEN), pEGFP, and pET (where BL21, a strain expressing T7 RNA polymerase, is preferably used as the host).

Furthermore, the vector may comprise a signal sequence for polypeptide secretion. When producing proteins into the periplasm of *E. coli,* the pelB signal sequence (Lei, S. P. et al. J. Bacteriol. 169:4379 (1987)) may be used as a signal sequence for protein secretion. For example, calcium chloride methods or electroporation methods may be used to introduce the vector into a host cell.

In addition to E. coli, expression vectors derived from mammals (e.g., pCDNA3 (Invitrogen), pEGF-BOS (Nucleic Acids Res. (1990) 18(17):5322), pEF, pCDM8), insect cells (e.g., "Bac-to-BAC baculovirus expression system" (GIBCO-BRL), pBacPAK8), plants (e.g., pMH1, pMH2), animal viruses (e.g., pHSV, pMV, pAdexLcw), retroviruses (e.g., pZIPneo), yeasts (e.g., "Pichia Expression Kit" (Invitrogen), pNV11, SP-Q01), and *Bacillus subtilis* (e.g., pPL608, pKTH50) may also be used as a vector for producing a polypeptide of the present invention.

In order to express proteins in animal cells such as CHO, COS, and NIH3T3 cells, the vector preferably has a promoter necessary for expression in such cells, for example, an SV40 promoter (Mulligan et al. (1979) Nature 277:108), MMLV-LTR promoter, EFlocpromoter (Mizushima et al. (1990) Nucleic Acids Res. 18:5322), CMV promoter, etc.). It is even more preferable that the vector also carry a marker gene for selecting transformants (for example, a drug-resistance gene enabling selection by a drug, such as neomycin and G418). Examples of vectors with such characteristics include pMAM, pDR2, pBK-RSV, pBK-CMV, pOPRSV, pOP 13, and such.

In addition, to stably express a gene and amplify the gene copy number in cells, CHO cells having a defective nucleic acid synthesis pathway can be introduced with a vector containing a DHFR gene (for example, pCHOI) to compensate for the defect, and the copy number may be amplified using methotrexate (MTX). Alternatively, a COS cell, which carries an SV40 T antigen-expressing gene on its chromosome, can be transformed with a vector containing the SV40 replication origin (for example, pcD) for transient gene expression. The replication origin may be derived from polyoma viruses, adenoviruses, bovine papilloma viruses (BPV), and such. Furthermore, to increase the gene copy number in host cells, the expression vector may contain, as a selection marker, an aminoglycoside transferase (APH) gene, thymidine kinase (TK) gene, *E. coli* xanthine guanine phosphoribosyl transferase (Ecogpt) gene, dihydrofolate reductase (dhfr) gene, and such.

Methods for expressing the DNAs of this invention in the bodies of animals include methods of incorporating the DNAs of this invention into appropriate vectors and introducing them into living bodies by, for example, a retrovirus method, liposome method, cationic liposome method, or adenovirus method. The vectors that are used include adenovirus vectors (for example, pAdexlcw), and retrovirus vectors (for example, pZIPneo), but are not limited thereto. General genetic manipulations such as inserting the DNAs of this invention into vectors can be performed according to conventional methods (Molecular Cloning, 5.61-5.63). Administration to living bodies can be carried out by *ex vivo* method or *in vivo* methods.

Furthermore, the present invention provides host cells into which a vector of this invention is introduced. The host cells into which a vector of this invention is introduced are not particularly limited; for example, E. coli and various animal cells are available for this purpose. The host cells of this invention may be used, for example, as production systems to produce and express the antibodies of the present invention. *In vitro* and *in vivo* production systems are available for polypeptide production systems. Production systems that use eukaryotic cells or prokaryotic cells are examples of *in vitro* production systems.

Eukaryotic cells that can be used include, for example, animal cells, plant cells, and fungal cells. Known animal cells include: mammalian cells, for example, CHO (J. Exp. Med. (1995)108, 945), COS, 3T3, myeloma, BHK (baby hamster kidney), HeLa, Vero, amphibian cells such as *Xenopus laevis* oocytes (Valle, et al. (1981) Nature 291, 358-340), or insect cells (e.g., Sf9, Sf21, and Tn5). CHO cells in which the DHFR gene has been deleted, such as dhfr-CHO (Proc. Natl. Acad. Sci. USA (1980) 77, 4216-4220) and CHO K-1 (Proc. Natl. Acad. Sci. USA (1968) 60, 1275), are particularly preferable for use as CHO cells. Of the animal cells, CHO cells are particularly favorable for large-scale expression. Vectors can be introduced into a host cell by, for example, calcium phosphate methods, DEAE-dextran methods, methods using cationic liposome DOTAP (Boehringer-Mannheim), electroporation methods, lipofection methods, etc.

Plant cells include, for example, Nicotiana tabacum-derived cells known as polypeptide production systems. Calluses may be cultured from these cells. Known fungal cells include yeast cells, for example, the genus Saccharomyces, such as Saccharomyces cerevisiae; and filamentous fungi, for example, the genus Aspergillus such as Aspergillus niger.

Bacterial cells can be used in prokaryotic production systems. Examples of bacterial cells include E. coli (for example, JM109, DH5α, HB101 and such); and Bacillus subtilis.

Antibodies can be obtained by transforming the cells with a polynucleotide of interest, then culturing these transformants *in vitro.* Transformants can be cultured using known methods. For example, DMEM, MEM, RPMI 1640, or IMDM may be used as the culture medium for animal cells, and may be used with or without serum supplements such as fetal calf serum (FCS). Serum-free cultures are also acceptable. The preferred pH is about 6 to 8 over the course of culturing. Incubation is typically carried out at a temperature of about 30 to 40°C for about 15 to 200 hours. Medium is exchanged, aerated, or stirred, as necessary.

On the other hand, production systems using animal or plant hosts may be used as systems for producing polypeptides *in vivo.* For example, a DNA of interest may be introduced into an animal or plant, and the polypeptide produced in the body of the animal or plant is then recovered. The "hosts" of the present invention include such animals and plants.

When using animals, there are production systems using mammals or insects. Mammals such as goats, pigs, sheep, mice, and cattle may be used (Vicki Glaser SPECTRUM Biotechnology Applications (1993)). Alternatively, the mammals may be transgenic animals.

For example, a DNA of interest may be prepared as a fusion gene with a gene encoding a polypeptide specifically produced in milk, such as the goat β-casein gene. DNA fragments containing the fusion gene are injected into goat embryos, which are then introduced back to female goats. The desired antibody can then be obtained from milk produced by the transgenic goats born from the goats that received the embryos, or from their offspring. Appropriate hormones may be administered to increase the volume of milk containing the polypeptide produced by the transgenic goats (Ebert, K.M. et al., Bio/Technology 12, 699-702 (1994)).

Insects, such as silkworms, may also be used. Baculoviruses carrying a DNA of interest can be used to infect silkworms, and the antibody of interest can be obtained from their body fluids (Susumu, M. et al., Nature 315, 592-594 (1985)).

When using plants, tobacco can be used, for example. When tobacco is used, a DNA of interest may be inserted into a plant expression vector, for example, pMON 530, and then the vector may be introduced into a bacterium, such as Agrobacterium tumefaciens. The bacteria are then used to infect tobacco such as Nicotiana tabacum, and the desired polypeptides are recovered from the leaves (Julian K.-C. Ma et al., Eur. J. Immunol. 24,131-138 (1994)).

The resulting antibodies of this invention may be isolated from the inside or outside (such as the medium) of host cells, and purified as substantially pure and homogenous antibodies. Any standard method for isolating and purifying antibodies may be used, and methods are not limited to any specific method. Antibodies may be isolated and purified by selecting an appropriate combination of, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, recrystallization, and others.

Chromatography includes, for example, affinity chromatography, ion exchange chromatography, hydrophobic chromatography, gel filtration, reverse-phase chromatography, and adsorption chromatography (Strategies for Protein Purification and Characterization: A Laboratory Course Manual. Ed Daniel R. Marshak et al., Cold Spring Harbor Laboratory Press, 1996). These chromatographies can be carried out using liquid phase chromatographies such as HPLC and FPLC. The present invention also includes antibodies that are highly purified using these purification methods.

In the present invention, the antigen-binding activity of antibodies (Antibodies A Laboratory Manual. Ed Harlow, David Lane, Cold Spring Harbor Laboratory, 1988) can be measured using well known techniques. For example, ELISA (enzyme linked immunosorbent assay), EIA (enzyme immunoassay), RIA (radioimmunoassay), or fluoroimmunoassay may be used.

In the present invention, whether or not the antibodies of this invention induce cell death in non-adherent cells can be determined from whether cell death is induced in Jurkat cells or ARH77 cells, as in the Examples. Whether or not the antibodies induce cell death in adhesion cells can be determined from whether cell death is induced in HeLa cells, as in the Examples.

Furthermore, the present invention provides cell death-inducing agents or cell growth-suppressing agents which comprise minibodies or 2D7 antibodies of this invention as active ingredients. The cell death-inducing activity of the minibodies or 2D7 antibodies in this invention is considered to have a particularly large effect on activated T cells or B cells; therefore, it is considered to be particularly effective for treatment and prevention of tumors such as cancer (particularly blood tumors), and autoimmune diseases. Accordingly, the present invention provides methods of treatment and prevention of tumors such as cancer (particularly blood tumors), and autoimmune diseases that use the minibodies or 2D7 antibodies of this invention. When using 2D7 antibodies whose molecular weight has not been reduced as active ingredients, they are preferably cross-linked with an anti-IgG antibody and such.

The above-mentioned antibodies can also be used as conjugate antibodies, after linking to various reagents. Examples of such reagents include chemotherapy reagents, radioactive substances, and toxins. Such conjugate antibodies can be produced by known methods (US5057313, and US5156840).

The above-mentioned pharmaceutical agents can be directly administered to patients, or administered as pharmaceutical compositions formulated by known pharmaceutical methods. For example, they may be administered orally, as tablets, capsules, elixirs, or microcapsules, sugar-coated as necessary; or parenterally, in the form of injections of sterile solution or suspensions prepared with water or other pharmaceutically acceptable liquids. For example, they may be formulated by appropriately combining them with pharmaceutically acceptable carriers or media, more specifically, sterilized water or physiological saline solutions, vegetable oils, emulsifiers, suspending agents, surfactants, stabilizers, flavoring agents, excipients, vehicles, preservatives, binding agents, and such, and mixing them at a unit dosage form required for generally accepted pharmaceutical practice. The amount of active ingredient in the formulation is such that appropriate doses within indicated ranges are achieved.

Additives that can be mixed into tablets and capsules include, for example, binding agents such as gelatin, cornstarch, tragacanth gum, and gum arabic; excipients such as crystalline cellulose; swelling agents such as cornstarch, gelatin, alginic acid; lubricants such as magnesium stearate; sweeteners such as sucrose, lactose, or saccharine; and flavoring agents such as peppermint and *Gaultheria adenothrix* oils, or cherry. When the unit dosage form is a capsule, liquid carriers, such as oils and fats, can be further included in the above-indicated materials. Sterile compositions to be injected can be formulated using a vehicle such as distilled water used for injection, according to standard protocols.

Aqueous solutions used for injections include, for example, physiological saline and isotonic solutions comprising glucose or other adjunctive agents such as D-sorbitol, D-mannose, D-mannitol, and sodium chloride. They may also be combined with appropriate solubilizing agents, such as alcohol, and specifically, ethanol, polyalcohol such as propylene glycol or polyethylene glycol, or non-ionic detergent such as polysorbate 80™ or HCO-50, as necessary.

Oil solutions include sesame oils and soybean oils, and can be combined with solubilizing agents such as benzyl benzoate or benzyl alcohol. Injection solutions may also be formulated with buffers, for example, phosphate buffers or sodium acetate buffers; analgesics, for example, procaine hydrochloride; stabilizers, for example, benzyl alcohol or phenol; or anti-oxidants. The prepared injections are typically aliquoted into appropriate ampules.

Administration to patients may be performed, for example by intra-arterial injection, intravenous injection, or subcutaneous injection, alternatively by intranasal, transbronchial, intramuscular, transdermal, or oral administration using methods well known to those skilled in the art. Doses vary depending on the body weight and age of the patient, method of administration and such; nevertheless, those skilled in the art can appropriately select suitable doses. Furthermore, if a compound can be encoded by a DNA, the DNA may be incorporated into a gene therapy vector to carry out gene therapy. Doses and administration methods vary depending on the body weight, age, and symptoms of patients, but, again, they can be appropriately selected by those skilled in the art.

A single dose of a pharmaceutical agent of this invention varies depending on the target of administration, the target organ, symptoms, and administration method. However, the usual dose for an adult (presuming a body weight of 60 kg) in the form of an injection is approximately 0.1 to 1000 mg, preferably approximately 1.0 to 50 mg, and more preferably approximately 1.0 to 20 mg per day, for example.

When administered parenterally, a single dose varies depending on the target of administration, the target organ, symptoms, and administration method. However, when in the form of an injection to an adult (presuming a body weight of 60 kg), usually it is considered advantageous to intravenously administer, for example, a single dose of about 0.01 to 30 mg, preferably about 0.1 to 20 mg, and more preferably about 0.1 to 10 mg per day. For other animals, a converted amount based on the amount for a body weight of 60 kg, or a converted amount based on the amount for a body surface area can be administered.

### Brief Description of the Drawings

Fig. 1 shows the adaptors used to produce the pMX2 vector. The bold letters indicate BstXI recognition sequences.
Fig. 2A and Fig. 2B show 2D7 antigen expression in cell lines. Each cell type was stained with 2D7 antibody and the expression examined. (Solid line: no primary antibody; dotted line: 2D7 antibody)
Fig. 3 is a set of photographs showing the results of immunoprecipitation using the 2D7 antibody. NIH3T3, RPMI 8226, and U266 cells were solubilized, immunoprecipitation was performed with the 2D7 antibody, anti-BST-1 antibody (control), or protein G itself, and the proteins were detected by silver staining. In RPMI 8226 and U266, a molecule of approximately 12 KD (arrow), which is specifically precipitated by the 2D7 antibody, was detected. This band was cut out and peptide sequenced, and thus found to be β2-microglobulin.
Fig. 4 shows flow diagrams for screening. Separation into pools, preparation of DNA, packaging into virus, infection of 3T3 cells, and screening using FACS were performed in one span (Fig. 4A). By the end of the fourth screening, the library was narrowed down to approximately 20 clones. In the fifth screening, 64 colonies were individually inoculated into a 96-well plate, pools were formed using the vertical and horizontal rows, and then screened. As a result, the library was narrowed down to twelve candidate clones (Fig. 4B).
Fig. 5 shows the results of screening using FACS. Fig. 5A shows the results of the second screening, Fig. 5B shows the results of the third screening, and Fig. 5C shows the results of the fourth screening. NIH3T3 cells were infected with retroviruses prepared from each pool, and three days later the cells were stained with the 2D7 antibody. The clones were narrowed down by gradually reducing the pool size of each screening.
Fig. 6 shows the results of screening using FACS. Fig. 6A shows the results of the fifth screening, and Fig. 6B shows the result of the final screening. As a result of the fifth screening, positive clones were found in rows 3, 4, 6, and 8, and in rows E, F, and G. As a result of screening the twelve candidate clones, positive clones were found in row E at 6E . When the nucleotide sequence of this 6E was analyzed, it was found to encode HLA classI A*6802.
Fig. 7 is a graph and a set of photographs showing the influence on cells of the addition of 2D7 antibody. 2D7 antibody (10 µg/ml) was added, and the number of viable cells was determined 48 hours later. Hardly any change in cell growth was observed, even after 2D7 antibody was added (Fig. 7A). K562 cells (Fig. 7B), Jurkat cells (Fig. 7C), and RPMI8226 cells (Fig. 7D) were each observed 24 hours after 2D7 antibody addition. The 2D7 antibody induced aggregation of Jurkat cells.
Fig. 8 is a set of photographs showing cell death induction due to cross-linking of the 2D7 antibody. Each combination of the 2D7 antibody with anti-mouse IgG was made to act on Jurkat cells, and the cell nuclei were stained 48 hours later. Nuclear fragmentation due to cell death was observed when the 2D7 antibody and anti-mouse IgG acted on cells simultaneously.
Fig. 9 shows a 2D7 diabody (2D7DB) sequence. The nucleotide sequences in the figure is shown in SEQ ID NO: 3, and the amino acid sequence is shown in SEQ ID NO: 4.
Fig. 10A and Fig. 10B show a 2D7 diabody structure. Fig. 10C is a photograph showing 2D7 diabody transient expression in COS7 cells.
Fig. 11A and Fig. 11B show the cytotoxic activity of 2D7DB transiently expressed in COS7.
Fig. 12 shows the cytotoxic activity of 2D7DB transiently expressed in COS7. K562 cells (Fig. 12A) and Jurkat cells (Fig. 12B) were used.
Fig. 13 shows the cytotoxic activity of 2D7DB transiently expressed in COS7. RPMI 8226 cells (Fig. 13A), IL-KM3 cells (Fig. 13B), U266 cells (Fig. 13C), and ARH77 cells (Fig. 13D) were used.
Fig. 14 is a graph showing the growth-suppressing effect of purified 2D7DB.
Fig. 15 shows cell death induction by purified 2D7DB, 48 hours after induction. ARH77 cells (Fig. 15A), Jurkat cells (Fig. 15B), K562 cells (Fig. 15C), and HeLa cells (Fig. 15D) were used.
Fig. 16 shows cell death induction by purified 2D7DB, 48 hours after induction. U266 cells (Fig. 16A), and IL-KM3 cells (Fig. 16B) were used for the study.
Fig. 17 shows a time course of cell death induction by 2D7DB (2 µg/ml). Cell death induction was investigated at 12 through to 38 hours. ARH77 cells (Fig. 17A) and Jurkat cells (Fig. 17B) were used.
Fig. 18 shows a time course of cell death induction by 2D7DB (2 µg/ml). Cell death induction was investigated at three through to six hours. ARH77 cells (Fig. 18A) and Jurkat cells (Fig. 18B) were used.
Fig. 19 shows the effect of Z-VAD-FMK on cell death due to 2D7DB. The study was performed using ARH77 cells 16 hours after induction.
Fig. 20 shows the effect of Z-VAD-FMK on cell death due to 2D7DB. The study was performed using Jurkat cells 16 hours after induction.
Fig. 21 is a set of photographs showing that cell death due to 2D7DB is not accompanied by DNA fragmentation. The study was performed 24 hours after cell death induction.
Fig. 22 shows the results of investigating the effect of cytochalasin D on the cell death-inducing activity of 2D7DB. By pre-treating ARH77 cells with cytochalasin D, which is an actin-polymerization inhibitor, the cells showed resistance to 2D7DB-induced cell death.
Fig. 23 is a set of photographs showing the results of immunostaining to investigate the state of the intracellular actin and nuclei. After reacting ARH77 cells under the conditions described in the figure, actin was detected using anti-actin antibody (red), and cell nuclei were detected using Hoechst 33258 (blue). Actin was absent in cells treated with 2D7DB.
Fig. 24 shows that 2D7DB suppresses an increase in human IgG (hIgG) concentration in serum in a mouse model of human myeloma. The data shows the average + SEM. There was a significant difference (*: p<0.05) between the vehicle-administered group and the 2D7DB-administered group, according to unpaired t-tests.
Fig. 25 shows that 2D7DB has a life-prolonging effect in a mouse model of human myeloma. There was a significant difference (*: p<0.05) between the vehicle-administered group and the 2D7DB-administered group, according to generalized Wilcoxon tests.
Fig. 26 shows analyses of the action of 2D7DB on PBMC. PHA-M (Fig. 26A), ConA (Fig. 26B), and SAC (Fig. 26C) were used as mitogens. Fig. 26D shows the results in the absence of a mitogen, and Fig. 26E shows the results of a positive control (ARH77). The results shown are, from the top, those of no 2D7DB addition, three-hour addition, and 24-hour addition.

### Best Mode for Carrying out the Invention

Herein below, the present invention is specifically described using Examples; however, it should not to be construed as being limited thereto.

### [1] Cell lines

Human myeloma cell lines (RPMI8226, K562, and ARH77), human T-cell leukemia cell line (Jurkat), FDC-P1, HCI-16, and 2D7 hybridoma cell line (from University of Tokushima) were cultured in RPMI1640 medium (GIBCO BRL) supplemented with 10% fetal calf serum (FCS). Human myeloma cell lines (IL-KM3 and U266) were individually cultured in the same medium supplemented with 2 ng/ml of IL-6 (R & D), and Ba/F3 was cultured in the same medium supplemented with 2 ng/ml of IL-3 (R & D). COS7, 293T, HeLa, NIH3T3, and BOSC23 were cultured in DMEM medium (GIBCO BRL) supplemented with 10% FCS, and CHO was cultured in α-MEM medium (GIBCO BRL) supplemented with 5% FCS or 10% FCS.

### [2] Production of pMX2 vectors

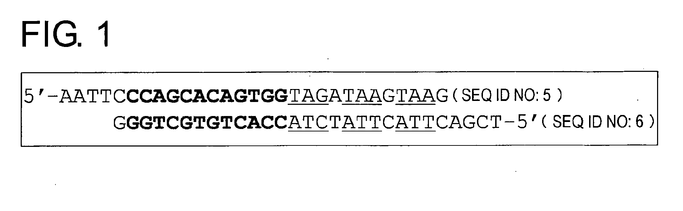

The GFP gene region of the retrovirus vector, pMX-GFP, which packages the GFP gene into the virus particle, was cut out and removed using EcoRI-SaII. The adaptor, which comprised a BstXI site in its sequence (Fig. 1) (and was synthesized with anABI DNA synthesizer, then annealed *in vitro* before use), was inserted into this region, forming pMX2.

### [3] Production of cDNA libraries

Total RNA was purified from RPMI8226 cells by standard methods using TRIzol (GIBCO BRL). Furthermore, the mRNAs were purified from 200 µg of this total RNA, using a µMACS mRNA Isolation kit (Miltenyi Biotec) according to the manufacturer's instructions. The cDNAs were synthesized using random hexamers (SuperScript Choice System for cDNA Synthesis; Invitrogen) with 3.6 µg of mRNA as template, and then a BstXI adaptor (Invitrogen) was linked to both ends. This cDNA was inserted into the pMX2 vector cleaved with BstXI, and was introduced into ELECTRO MAX DH10B (GIBCO BRL) by electroporation (2.5 KV, 200 Ω, 25 µF). After adding 1 ml of SOC, the vectors were then incubated at 37°C for one hour, 1 ml of 40% glycerol/LB+Amp was added. A portion of the culture was used to check the titer and the remainder was stored at -80°C. The obtained library was plated at 200 µl/well (7% DMSO/LB+Amp) into two 96-well plates, so that each well contained 1000 clones. These were cultured overnight at 37°C. Four wells (4000 clones) from this plate were combined and placed into an ampicillin-containing LB medium (4 ml). This was defined as one pool, the rest of the wells were treated similarly. Ultimately, 24 pools were prepared from a single plate. After incubating each pool overnight at 37°C, DNAs were prepared (QIAGEN) and used for transfection into packaging cells. The plates used for inoculation were stored at -80°C until use in secondary screening.

### [4] Purification of antibodies

0.5 ml of ascites, sent from University of Tokushima, was adsorbed to a Protein A Hi Trap Affinity column (Amersham Pharmacia). The IgG fraction was then eluted using 0.1 M sodium citrate, pH3.0, and the 2D7 antibody was collected. This was concentrated using Centricon (YM-10; Millipore), and the buffer was exchanged to PBS to ultimately yield a total of 5.34 mg of antibody. This was separated into aliquots and stored at -20°C (concentration: 0.89 µg/µL).

### [5] FACS

Adherent cells were detached using 1 mM EDTA/PBS, and non-adherent cells were collected by centrifugation, then suspended in FACS buffer (2.5% FCS, 0.02% NaN₃/PBS). These cells were left to stand on ice for one hour in a buffer (5% FCS/PBS) containing 2D7 antibody (final concentration 10 µg/ml). These were then washed with FACS buffer, reacted in a solution of FITC-anti-mouse IgG (Immunotech) (1:150, 50 µL FACS buffer) on ice for 30 minutes, washed twice with FACS buffer, and then analyzed using EPICS ELITE (COULTER).

### [6] Retrovirus infection

### (i) Retrovirus packaging

The day before transfection, 2ml of BOSC23 cells, which are retrovirus-packaging cells, were plated onto a 6-well plate at 6 x 10⁵ cells/well. Transfection was carried out by the following procedure: 1 µg of the plasmid DNA derived from each pool was mixed with 3 µL of FuGENE 6 Transfection Reagent (Roche), left to stand at room temperature for 20 minutes, and then added to the BOSC23 cell culture medium plated the day before. Cells were then cultured at 37°C for 48 hours, and the culture medium was collected. Dead cells were removed by centrifugation at 3000 rpm for five minutes, and the culture solution was then used as the virus solution.

### (ii) Virus infection

NIH3T3 cells plated onto 6-well plates (1 x 10⁵ cells/2ml/well) the day before virus infection. Then 1 ml of virus solution supplemented with 10 µg/ml of polybrene (hexadimethrine bromide; Sigma) was added to the wells and NIH3T3 cells were cultured for 24 hours. 1.5 ml of fresh medium was then added, the cells were cultured for another 48 hours, and gene expression was then analyzed using FACS.

### [7] Immunoprecipitation

Cells were lysed in a lysis buffer (0.5% Nonidet P-40, 10 mM Tris, pH 7.6, 150 mM NaCl, 5 mM EDTA, 1 mM phenylmethylsulfonyl fluoride, 5 µg/ml aprotinin), and the resulting solution was centrifuged to remove the insoluble proteins and obtain a cell lysate. 1 µg of 2D7 antibody was added, and incubated at 4°C for four hours. Magnetic protein G (BioMag) was then added, and this was incubated for another one hour. Subsequently, the immunoconjugate was washed three times with a lysis buffer, and then subjected to SDS-PAGE. This gel was silver stained (Daiichi Pure Chemicals) according to the attached instructions. For peptide sequencing, the gel on which SDS-PAGE was performed was transferred to ProBlott (Applied Biosystems), and this was stained for one minute with Coomassie blue staining solution (0.1% coomassie blue R-250 in 40% MetOH/1% acetic acid). After washing several times with 50% MetOH, the band of interest was cut out, washed five times with 1 ml of DDW, dried *in vacuo,* and then subjected to peptide sequencing.

### [8] Cell growth assay using the 2D7 antibody

Each type of cell was plated into a 96-well plate at 1 x 10⁶ cells/ml in the presence or absence of PMA (50 ng/ml; GIBCO BRL) and PHA(10 µl/ml; GIBCO BRL). This was cultured for 48 hours after subsequent addition (10 µg/ml) or no addition of the 2D7 antibody,. After culturing, morphological changes in the cells were observed under a microscope. The relative viable cell count was determined by adding WST-8 (viable cell count reagent SF; Nacalai Tesque), culturing at 37°C for two hours, and measuring OD₄₅₀.

### [9] Induction of cell death by cross-linking

Jurkat cells were plated on a 24-well plate at 8 x 10⁵cells/well, and 10 µg/ml of anti-mouse IgG (Fc) antibody (Cappel) was further added in the presence (5 µg/ml) or absence of 2D7 antibody. 48 hours later, the cells were collected, and after washing with PBS, methanol was added to a concentration of 70%, and this was left to stand at -20°C for 15 minutes. After washing the cells several times with FACS buffer, Hoechst 33258 was added at a concentration of 10 µg/ml, and this was incubated at room temperature for 30 minutes. The cells were washed again with FACS Buffer, and an aliquot of the cells was then placed on a slide glass to observe the state of the nuclei under a fluorescence microscope.

### [10] Cloning of the 2D7 variable region

Total RNA was purified from 2D7 hybridoma (provided by University of Tokushima) using TRIzol according to standard methods. Using 3 µg of this RNA as a template, cDNAs were synthesized using a SMART RACE cDNA Amplification kit (CLONTECH), according to the attached instructions. Using this cDNA as a template, the variable regions of the heavy chain and light chain were amplified by PCR using the following primers:
Heavy chain: 5'-CAGGGGCCAGTGGATAGACTGATG (SEQ ID NO: 7)
Light chain: 5'-GCTCACTGGATGGTGGGAAGATG (SEQ ID NO: 8)
The amplified cDNAs encoding each of variable regions were subcloned into pCR-TOPO vector (Invitrogen), and the nucleotide sequences (SEQ ID NOs: 1 and 2) were determined.

### [11] Production of 2D7 diabody expression vector

Plasmids, to which each of the variable region cDNAs were subcloned, were used as templates, and the variable regions of the heavy chain and light chain (VH and VL) were respective amplified using the primers below:
Heavy chain
   2D7DB-H1: 5'-CCTGAATTCCACCATGCGATGGAGCTGGATCTTTC (SEQ ID NO: 9)
   2D7DB-H2: 5'-AATTTGGCTACCGCCTCCACCTGAGGAGACTGTGAGAGTGGTGCCCT (SEQ ID NO: 10)
Light chain
   2D7DB-L1: 5'-TCCTCAGGTGGAGGCGGTAGCCAAATTGTTCTCACCCAGTCGCCAGC (SEQ ID NO: 11)

2D7DB-L2:
5'-ATTGCGGCCGCTTATCACTTATCGTCGTCATCCTTGTAGTCTTTTATCTCCAACTTTG
TCCCCGAGCC (SEQ ID NO: 12)

Each of the VH and VL cDNAs amplified by these primers were combined into one tube, and further subjected to PCR. Using the PCR products as templates, PCR was performed again, this time using 2D7DB-H1 and 2D7DB-L2 as primers, to synthesize cDNAwith VH and VL linked through a 5-mer linker (SEQ ID NO: 3). This cDNA was digested with EcoRI-NotI and inserted into the EcoRI-NotI gap of the animal cell expression vector, pCXND3. The nucleotide sequence was confirmed, completing the construction of the 2D7 diabody expression vector, pCXND3-2D7DB.

### [12] Transient expression in COS7 cells

2 µg of pCXND3-2D7DB, or of an empty vector used as a control, was mixed with 6 µL of transfection reagent (LT-1, MIRUS) according to the attached instructions, and this was added to COS7 cells (plated the day before into a 6-well plate at 1x 10⁵ cells/well) whose medium had been exchanged to a serum-free medium (OPTI-MEM, GIBCO BRL). Five hours later, 200 µL of serum was added, and this was cultured for two to three days. The medium was collected, and dead cells were removed by centrifugation. The culture supernatant was then used for an experiment to detect cytotoxic activity.

Expression of 2D7DB in the culture supernatant was confirmed by Western blotting. More specifically, equal amounts of 2x SDS-PAGE Sample buffer and culture supernatant were added. In addition, after lysing the cells by adding a lysis buffer (0.5% Nonidet P-40, 10 mM Tris, pH 7.6, 150 mM NaCl, 5 mM EDTA), insolubilized proteins were removed by centrifugation to prepare a cell lysate, and an equal amount of 2x SDS-PAGE Sample buffer was added to this. After performing SDS-PAGE on each sample, the gels were transferred to PVDF membranes, and expression of the 2D7 single chain was detected using anti-FLAG antibody.

### [13] Establishment of expression cell lines producing 2D7 diabody

20 µg of pCXND3-2D7DB, linearized by cleaving with PvuI, was introduced to CHO cells (DXB11 strain) by electroporation, as described below.

After washing the CHO cells twice with ice-cold PBS, they were suspended in PBS at 1x 10⁷ cells/ml. 20 µg of the above-mentioned plasmid was mixed into these cells, and this was electropulsed (1.5 KV, 25 µFD). The cells were diluted into appropriate fractions, plated on to a 10 cm dish, and cultured in the presence of G418 (GIBCO BRL) at a fmal concentration of 50 µg/ml. Approximately 30 clones were selected from the grown colonies, and the diabody expression levels in the culture supernatants were investigated by Western blotting. The clone with the highest expression level was expanded in a nucleic acid-free MEMα medium containing 5 nM MTX, and this was stocked as a high-producing cell line.

### [14] Large-scale purification of 2D7 diabodies

A subconfluent 2D7DB-producing CHO cell line in a T-125 flask was detached using Trypsin-EDTA, and then this was transferred to a roller bottle (250 ml of MEMα without nucleotide + 5% FCS). Four days later, the culture solution was removed, and the cells were washed twice with PBS. The medium was then exchanged to 250 ml of CHO-S-SFMII medium (GIBCO BRL) to produce a serum-free medium, cells were cultured for three days, and then the cell culture supernatant was collected. This was filtered and used for purification after removing the dead cells by centrifugation,.

Purification of single chain Fv was performed as follows: First, the collected culture supernatant was applied and adsorbed onto an anti-Flag M2 column. After washing with buffer A (50 mM Tris-HCl pH7.4, 150 mM NaCl, 0.01% Tween 20), single chain Fv was eluted with buffer B (100 mM Glycine pH3.5, 0.01% Tween 20). The collected sample was immediately neutralized with Tris-HCl pH8.0 so that the final concentration was 25 mM. This was then used for gel filtration purification by a Superdex 200HR (26/60) column. The dimer fraction of single chain Fv was collected in PBS containing 0.01 % Tween 20. A portion of the collected sample was subjected to SDS electrophoresis and silver staining to confirm that the protein of interest has been purified, and then this was concentrated to produce a purified 2D7 diabody preparation.

### [15] Cell death induction experiment using 2D7 diabody

Various blood cell lines were plated into 24-well plates at 2-5 x 10⁵ cells/well. Purified 2D7DB, or the culture supernatant of COS7 transiently expressing 2D7DB, was added and cell death was induced. When the culture supernatant of COS7 transiently expressing 2D7DB was used, the supernatant was added till its concentration was 50%. The amount of medium in each well was 0.8 to 1 ml/well. When stimulating Jurkat cells, Con A (WAKO) was added at the time of 2D7DB addition to a final concentration of 2 µg/ml.

Adherent cells (HeLa) were plated into a 6-well plate at 2x 10⁵ cells/well, and the cells were attached by culturing overnight. Subsequently, purified 2D7DB was added to the culture solution.

Several hours to several days after 2D7DB addition, the non-adherent cells were collected as they were, and adherent cells were collected after detaching the cells with 1 mM EDTA/PBS. The cells were then washed with ice-cold PBS, and labeled with Annexin V, which is an apoptosis marker, and with PI, which is a dead-cell marker, according to the attached instructions (TACS Annexin V-FITC Apoptosis Detection Kit, TREVIGEN Instructions). The proportion of stained cells was then measured using flow cytometry (EPICS ELITE, COULTER).

### [16] Cell death induction by Actinomycin D

Various blood cell lines were plated into 24-well plates at 2-5 x 10⁵ cells/well. To inhibit the initial stage of apoptosis, a caspase inhibitor (Z-VAD-FMK, Promega) was added at a final concentration of 50 µM, and after incubating for 2.5 hours, cell death was induced. For cell death induction by Actinomycin D, Actinomycin D (Sigma) was added at 1 µg/ml (Jurkat) or 5 µg/ml (ARH77), and for cell death induction by 2D7DB, 2 µg/ml of purified 2D7DB was added. Cells were collected 16 hours after cell death induction, and stained using Annexin V and PI.

### [17] Cell growth assay using 2D7 diabody

Each type of cells was plated into a 96-well plate at a cell concentration of 1-2 x 10⁴ cells/well. 2D7DB was added at an appropriate concentration, and the cell count was determined after three days of culturing. Viable cell count was determined using WST-8. More specifically, this reagent was added to the cells at 10 µl/well, and the cells were then cultured at 37°C for 1.5 hours. The relative viable cell count was determined by measuring the OD₄₅₀ using a spectrophotometer. The growth suppression rate was calculated from (1- (OD₄₅₀ of 2D7DB treated cells / OD₄₅₀ of 2D7DB untreated cells)) x 100.

### [18] Detection of DNA fragmentation

ARH77 and Jurkat cells were plated into a 6-well plate so that the cell concentration was 2 x 10⁶ cells/well, and cell death was induced by adding purified 2D7DB at a final concentration of 2 µg/ml, or Actinomycin D at a final concentration of 1 µg/ml (ARH77) or 5 µg/ml (Jurkat) to each well. The control was a well to which nothing was added. After culturing for 24 hours, the cells were collected, washed once with PBS, and then lysed in a lysis buffer (10 mM Tris pH7.5, 10 mM EDTA, 0.5% Triton X-100). This was followed by centrifugation to remove the insoluble proteins, and then the material was treated with RNase A and Proteinase K. A portion of this was then subjected to agarose gel electrophoresis to detect chromatin DNA fragmentation.

### [19] Inhibition of cell death induction by cytochalasin D

ARH77 cells were plated into a 24-well plate to achieve a cell concentration of 5 x 10⁵ cells/well, and cytochalasin D (Sigma) was added to a final concentration of 20 µg/ml. The control was a well to which ethanol alone was added. After culturing for one hour, purified 2D7DB was added at various concentrations (0, 200, 500, 1000 ng/ml), and culturing was continued for another four hours. Cells were then collected, and the proportion of dead cells was detected by staining with PI.

### [20] Immunostaining of 2D7DB-treated cells using anti-actin antibody

2D7DB was added at a concentration of 1 µg/ml to cytochalasin D-treated/-untreated ARH77 cells, and after culturing at 37°C for 15 minutes, the cells were adhered to a slide glass with a Cytospin. After immobilizing the cells by immersion in methanol for 15 minutes at -20°C, blocking was performed using a blocking buffer (3% BSA/PBS) at 4°C for one hour. This was then reacted with CY3-labeled anti-actin antibody (Sigma) diluted 100-fold in 1% BSA/PBS for one hour at room temperature, and then the cell nuclei were stained with Hoechst 33258. After washing several times with PBS, the cells were observed under a confocal laser scanning microscope (Olympus).

### [Example 1] Expression analysis of 2D7 antigen in each type of cell line

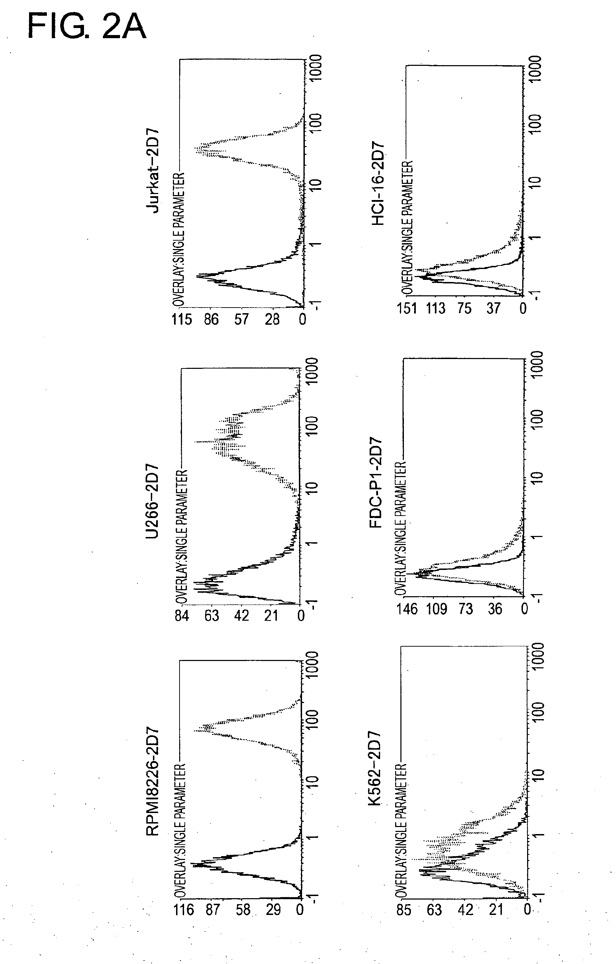

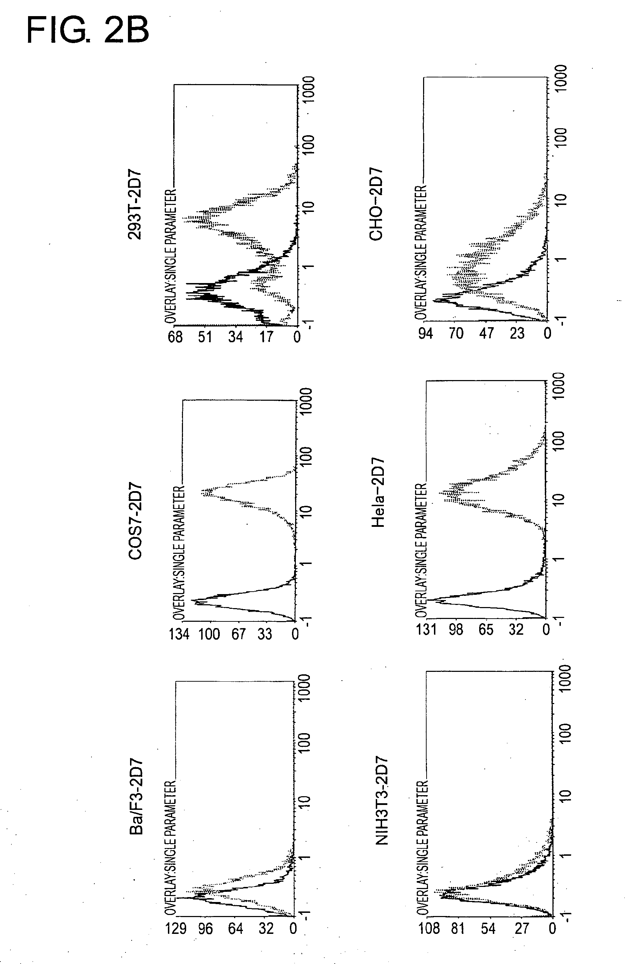

To determine the cell line that should become the source to produce a cDNA expression library and the cell line that should become the host, 2D7 antigen expression in each type of animal cell was analyzed using FACS (Fig. 2A and Fig. 2B). As a result, among human-derived blood cells, extremely strong expression of the 2D7 antigen was observed in lymphocytic tumor cell lines, RPMI8226, U266, and in Jurkat, but expression was found to be weak in K562. In Ba/F3, FDC-P1, and HCI-16, which are blood cells derived from mice, expression was very weak, perhaps due to differences between species. Of the adherent cells, expression was observed in COS7, 293T, and HeLa. Expression was hardly observed in mouse NIH3T3 cells.

From the expression patterns mentioned above, RPMI8226 cells were judged to be appropriate as a cDNA library source to be used for expression cloning, and NIH3T3 cells were determined to be appropriate as host cells to be used for screening, to which the expression library is transferred.

### [Example 2] Cloning of 2D7 antigen

### [1] Cloning from a protein

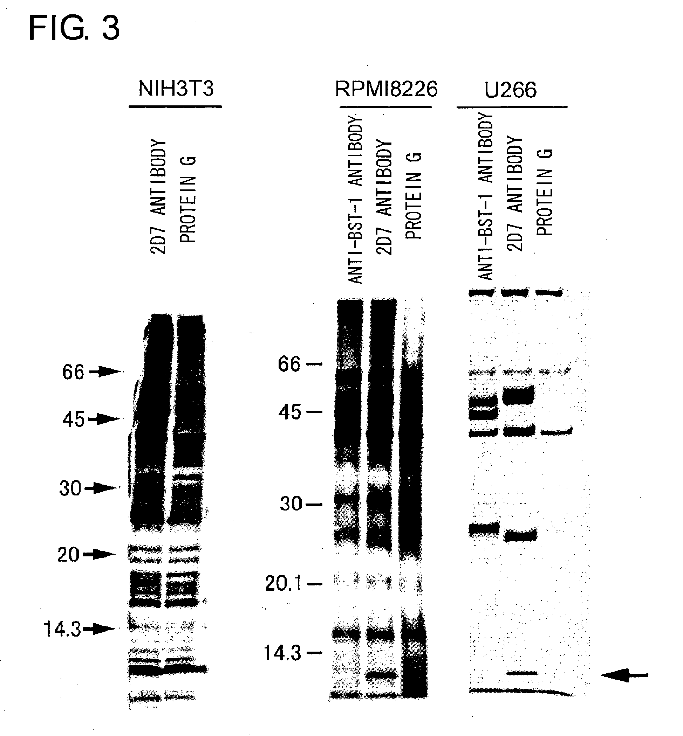

Cell lysates were prepared from RPMI8226 cells and U266 cells, which express the 2D7 antigen, and NIH3T3 cells, which do not express the 2D7 antigen, and immunoprecipitation was performed using the 2D7 antibody. As a result, a molecule (approximately 12 kD) that precipitates specifically in RPMI8226 and U266 cells was observed (Fig. 3). This molecule was not detected by Western blotting using the 2D7 antibody, but since it is at least reproducibly precipitated by the 2D7 antibody, it was strongly predicted to be the 2D7 antigen itself, or a molecule that co-precipitates with the 2D7 antigen.

Coomassie staining was performed on this band; it was then cut out and the peptides were sequenced. As a result, this 12 kD molecule was identified as β2 microglobulin (β2M). Since β2M is one of the class I MHC protein complexes that associate with HLA class I through non-covalent bonds, the 2D7 antibody is considered to have co-precipitated it as an HLA complex. HLA class I comprises the α1 and α2 domains required for antigen presentation, and the α3 domain which binds to β2M. Since the 2D7 antibody can co-precipitate the β2M molecule, it is anticipated that the 2D7 antibody will recognize the α1-α2 domains of HLA class I as an epitope.

### [2] Expression cloning of genes

cDNAs were synthesized using random hexamers from mRNAs purified from the 2D7 antigen-expressing cells, RPMI8226. These were inserted into a retrovirus vector, pMX2, and a retrovirus expression library was constructed. The library titer was investigated, and found to include a total of 6 x 10⁶ clones. Furthermore, the average cDNA length was found to be approximately 1.5 kb, arrived at by randomly selecting 24 clones from this library and investigating their insert size using colony PCR. Thus, the produced expression library was judged to be sufficient for use in expression cloning.

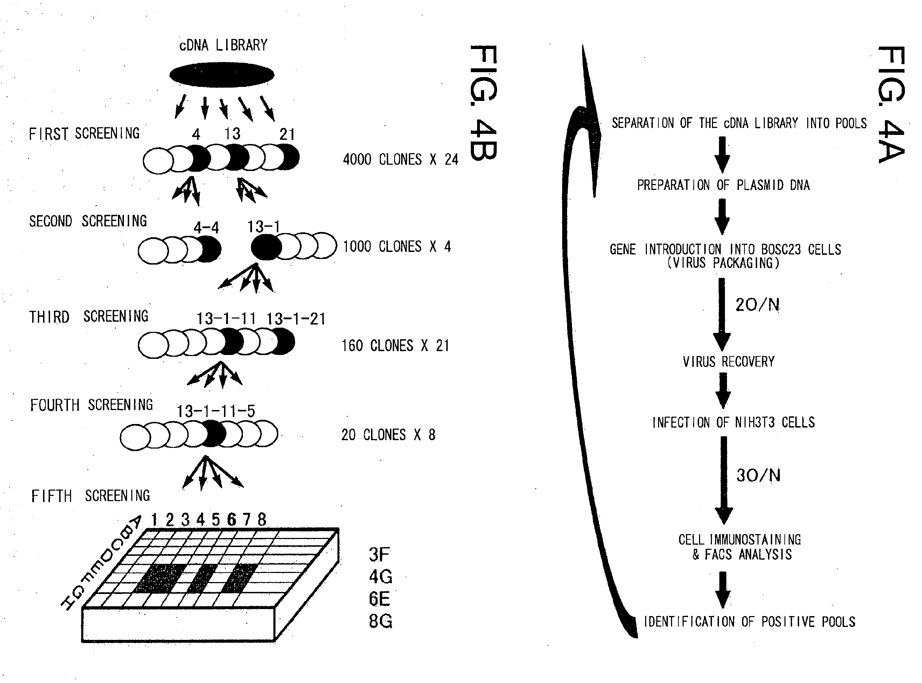

Fig. 4A and Fig. 4B show a flow diagram of the screening described below. In the first screening, 4000 independent clones were used in one pool, and 24 pools (corresponding to 96000 clones) were produced. The plasmids were packaged into retroviruses by transfecting each plasmid into BOSC23 cells. The resulting viruses derived from each pool were infected into NIH3T3 cells. Three days after infection, the cells were detached, and after staining with 2D7 antibody, expression analysis was performed using FACS. As a result, compared to NIH3T3 cells infected with viruses derived from an empty vector (control), 2D7-positive cells were found in 3 of the 24 pools (pools 4, 13, and 21).

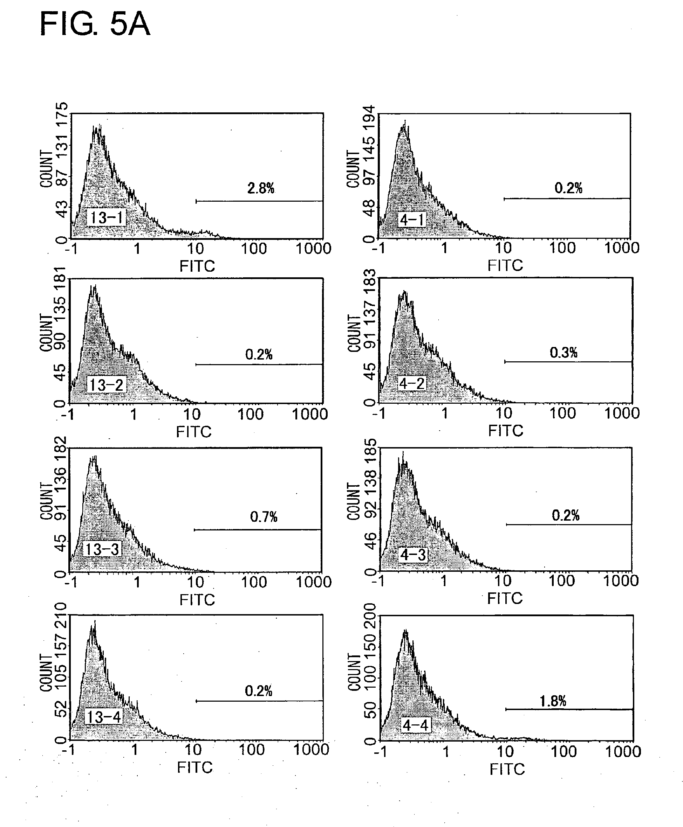

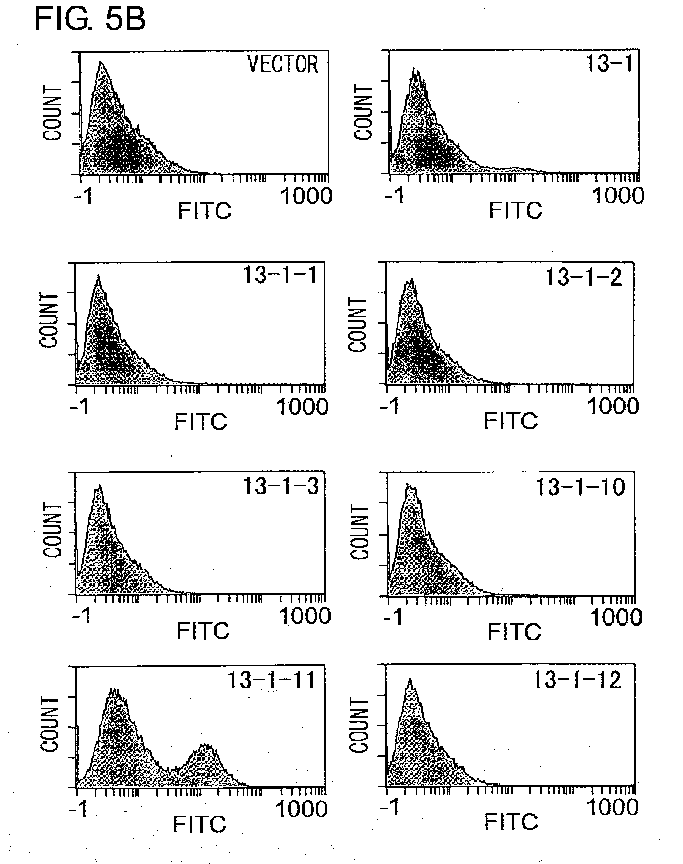

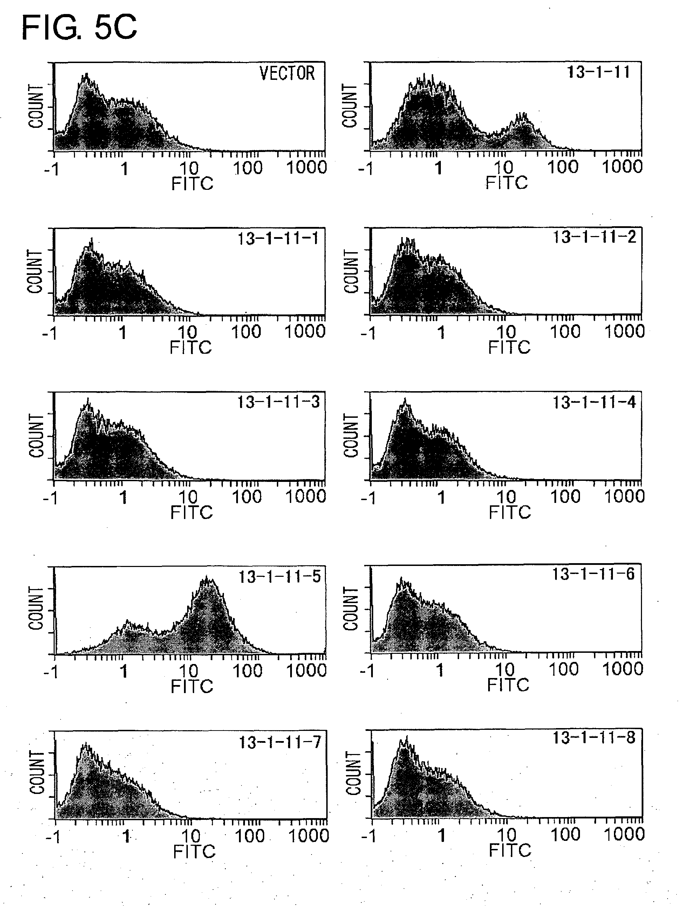

Next, pools 4 and 13, which showed positive results in the first screening, were divided into four pools each comprising 1000 independent clones, and a second screening was performed. As a result, a single clearly positive pool was found from each pool (Fig. 5A, pool 4-4, and pool 13-1). Pool 13-1 was further divided into 21 pools, each comprising 160 independent clones, to perform a third screening. Two positive pools (Fig. 5B, 13-1-11 and 13-1-21) were identified. Subsequently, pool 13-1-11 was divided into eight pools, each comprising 20 clones, to perform a fourth screening, and a positive pool (Fig. 5C, 13-1-11-5) was obtained.

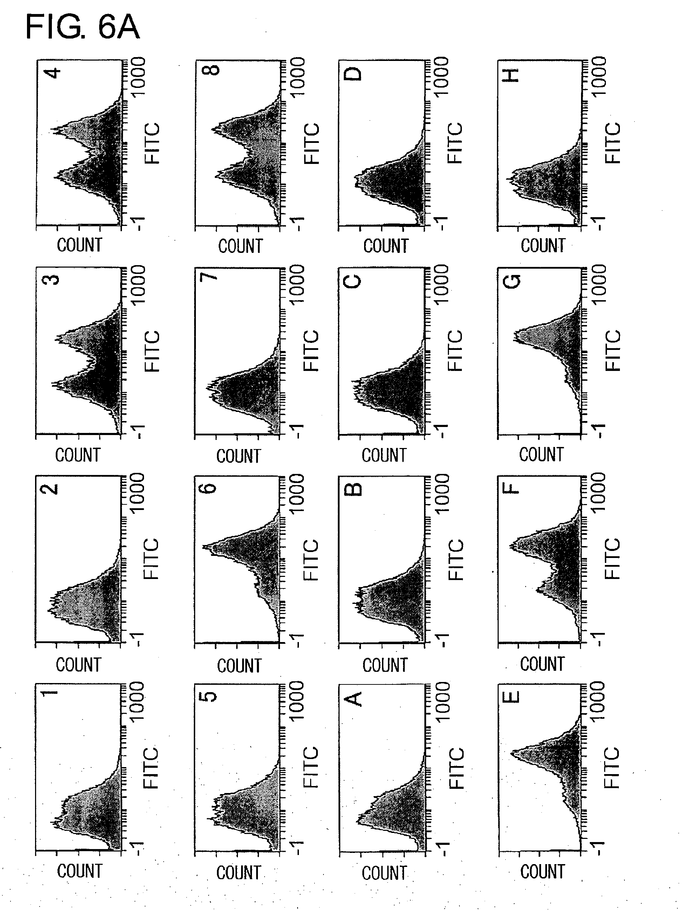

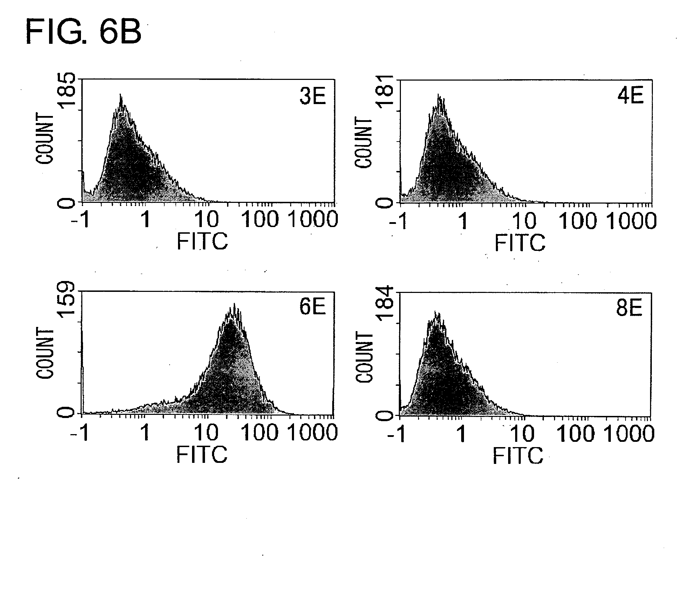

This pool was spread onto an LB plate, 64 colonies were picked one by one, and each of these were inoculated to one well of a 96-well plate. The eight clones in the vertical rows were taken as one pool to produce eight pools (1 to 8), and the eight clones in the horizontal rows were taken as one pool to produce eight pools (A to H), and a fifth screening was performed. As a result, pools 3, 4, 6, and 8, and pools E, F, and G were positive, thus narrowing down the positive candidate clones to twelve clones (Fig. 6A). FACS was performed on these twelve clones, and ultimately four positive clones (3F, 4G, 6E, and 8G) were identified as a single clone recognized by the 2D7 antibody (Fig. 6B).

As a result of reading the sequence of the insert portion of these clones, all four clones were found to be the full-length cDNA sequence of Human MHC class I HLA-A-6802.

HLA-A is classified into several dozen types of haplotypes. As a result of this cloning, the A*6802 haplotype of HLA class I was identified as a 2D7 antigen, but since the 2D7 antibody recognizes a wide variety of cells, the haplotype of HLA class I in the RPMI8226 cells that were used as the gene source just happened to be A*6802, and the 2D7 antibody was considered to be an antibody that recognizes any haplotype of HLA class I molecules.

### [Example 3] Examination of growth inhibitory effect

Several types of leukemia cell lines (K562, Jurkat, and RPMI8226) were used to investigate whether the 2D7 antibody has a cytocidal effect. The expression level of the 2D7 antigen in the three cell lines is: K562, weakly positive; Jurkat and RPMI8226, strongly positive.

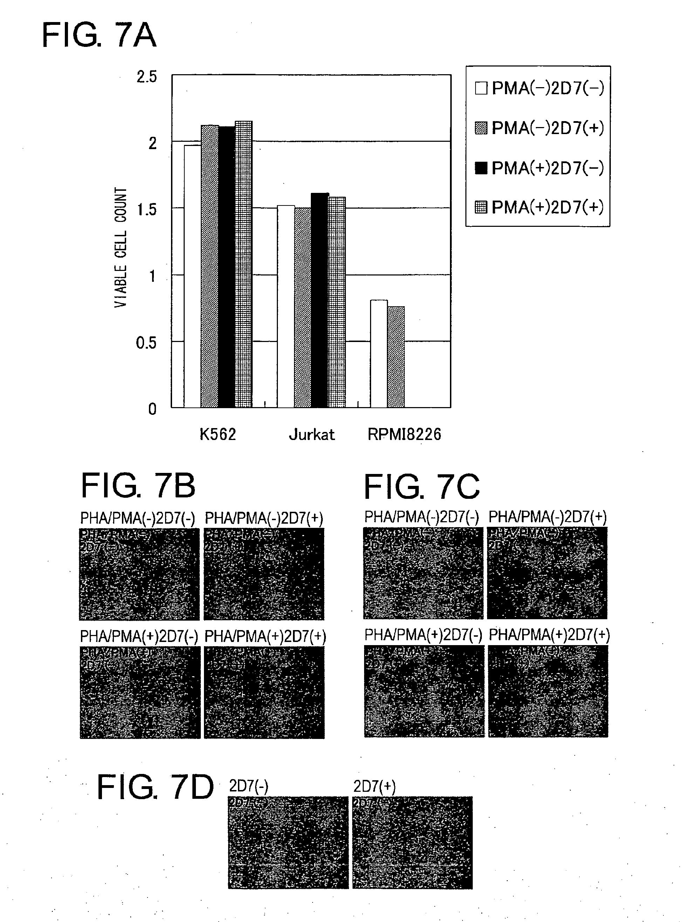

K562 and Jurkat cells were plated in the presence or absence of PHA and PMA, and 10 µg/ml of the 2D7 antibody was added thereto. On observing the cells 24 hours later, weakly 2D7-positive K562 cells did not show obvious differences in their morphology due to the presence or absence of the 2D7 antibody, however, addition of 2D7 antibody resulted in significant cell aggregation in Jurkat cells strongly expressing 2D7 (Fig. 7B and Fig. 7C). However, growth inhibition due to addition of the 2D7 antibody was not observed (Fig. 7A). Growth inhibition due to 2D7 in Jurkat cells activated by PHA and PMA stimulation was also not observed.

Unexpectedly, addition of 2D7 antibody did not have an obvious effect on the morphology and growth of the strongly 2D7-positive RPMI8226 cells (Fig. 7D).

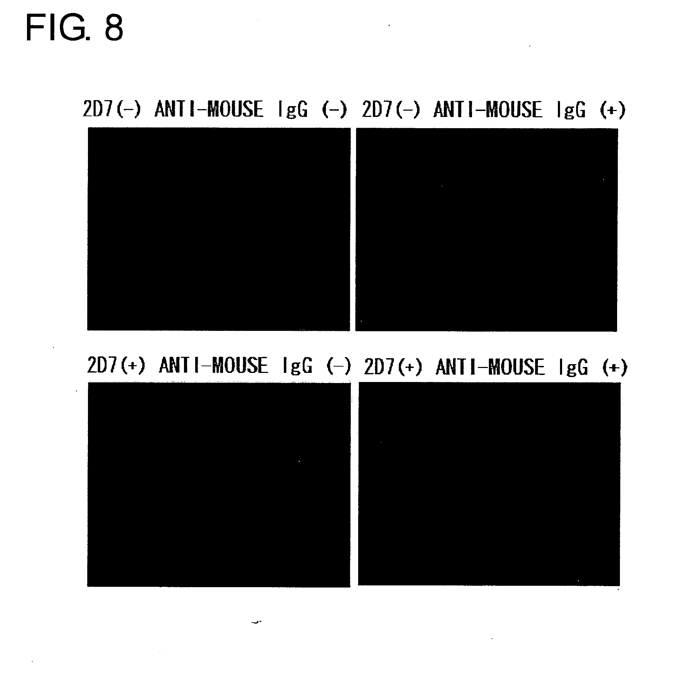

Next, it was examined whether cytocidal effects can be observed by adding anti-mouse IgG(Fc) antibody to 2D7 antibody, to cross-link the antibodies. Anti-mouse IgG was added to Jurkat cells, in the presence or absence of 2D7 antibody. The cells were cultured, and 48 hours later the cell nuclei were stained with Hoechst33258. Cells were observed for fragmentation of cell nuclei, which is characteristic of dead cells (Fig. 8). As a result, nuclear fragmentation was observed in Jurkat cells by further cross-linking 2D7 with an antibody, indicating that cell death was induced.

### [Example 4] Cloning of cDNA encoding the 2D7 antibody variable region, and the predicted diabody structure

Primers for the constant regions of the heavy chain and light chain of mouse IgG2b were produced, and DNA encoding the 2D7 variable region was cloned by 5'RACE method. The nucleotide sequences of the obtained PCR products are shown in SEQ ID NO: 1 and 2.

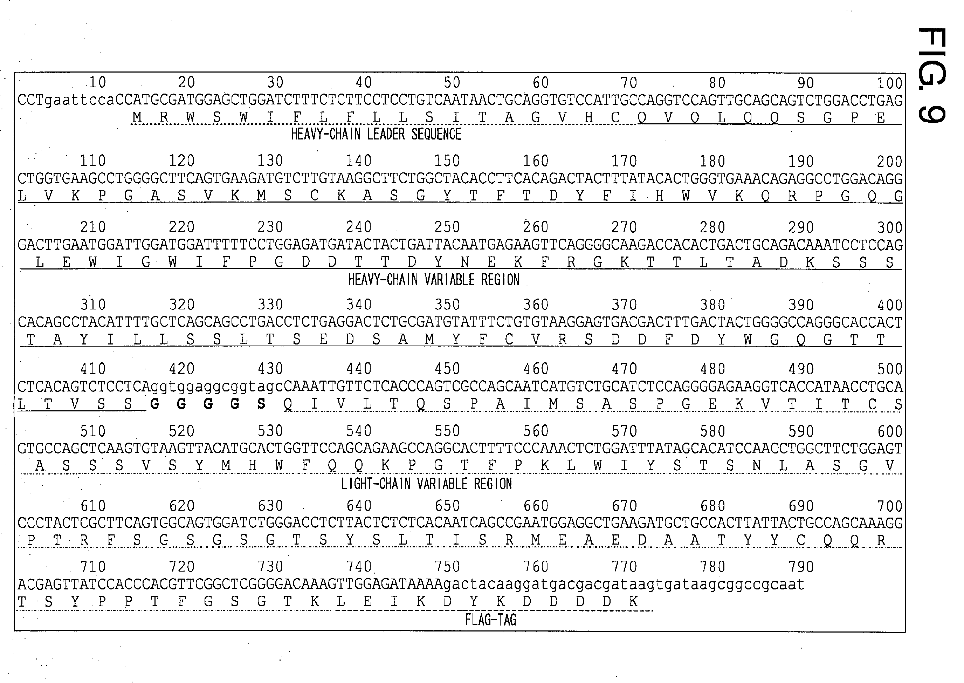

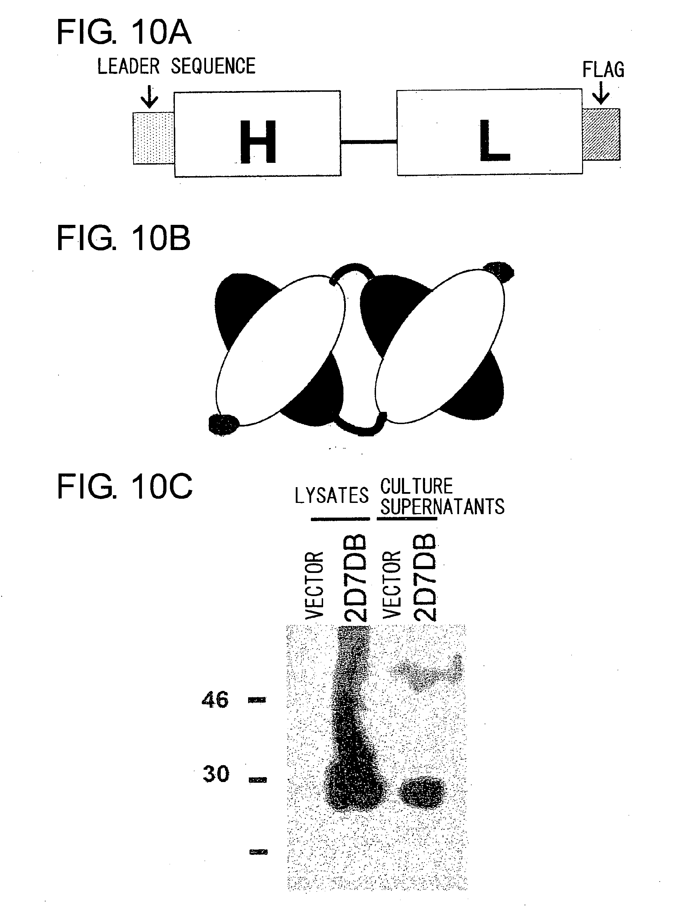

A single chain was then constructed based on these sequences. As shown in Fig. 9 and Fig. 10A, the 2D7 single chain is composed of the leader sequence of the heavy chain, the variable region of the heavy chain, and then across from a 5mer linker (GGGGS), the variable region of a light chain, followed by a cDNA (SEQ ID NO: 3) encoding a Flag-tag. Dimerization of this single chain may cause the 2D7 diabody to form the structure shown in Fig. 10B.

### [Example 5] Analysis of the cytotoxic activity of the 2D7 diabody

### (i) Cytotoxic activity of the 2D7 diabody transiently expressed in COS7

A 2D7 diabody expression vector was transfected into COS7 cells, and the culture supernatant was collected three days later. The culture supernatant and cell lysate were subjected to SDS-PAGE, and after performing Western blotting with an anti-Flag-tag antibody, a 2D7 single chain was found to be secreted in the culture supernatant (Fig. 10C).

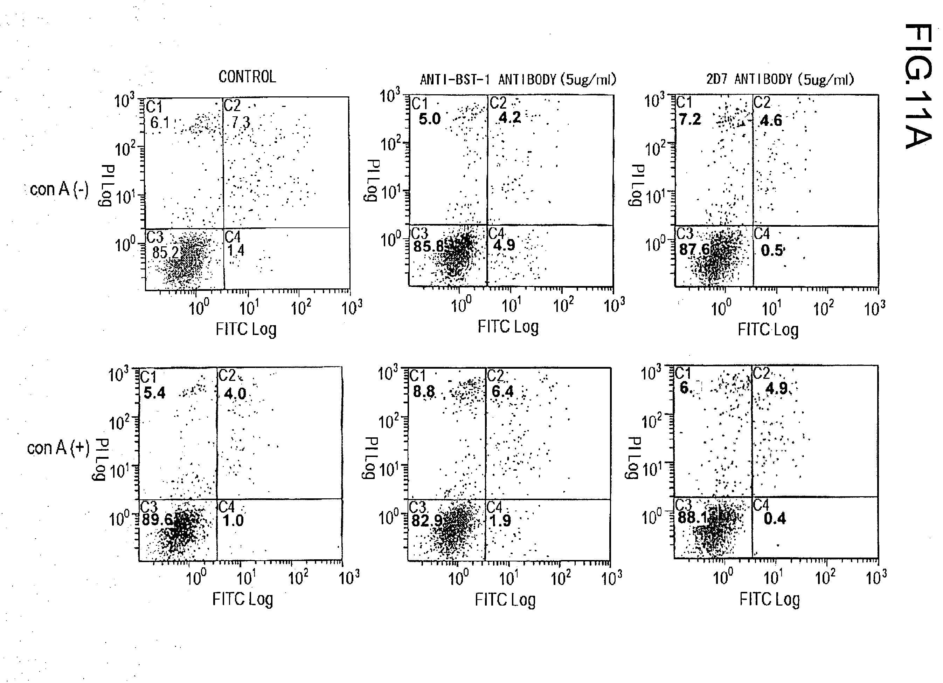

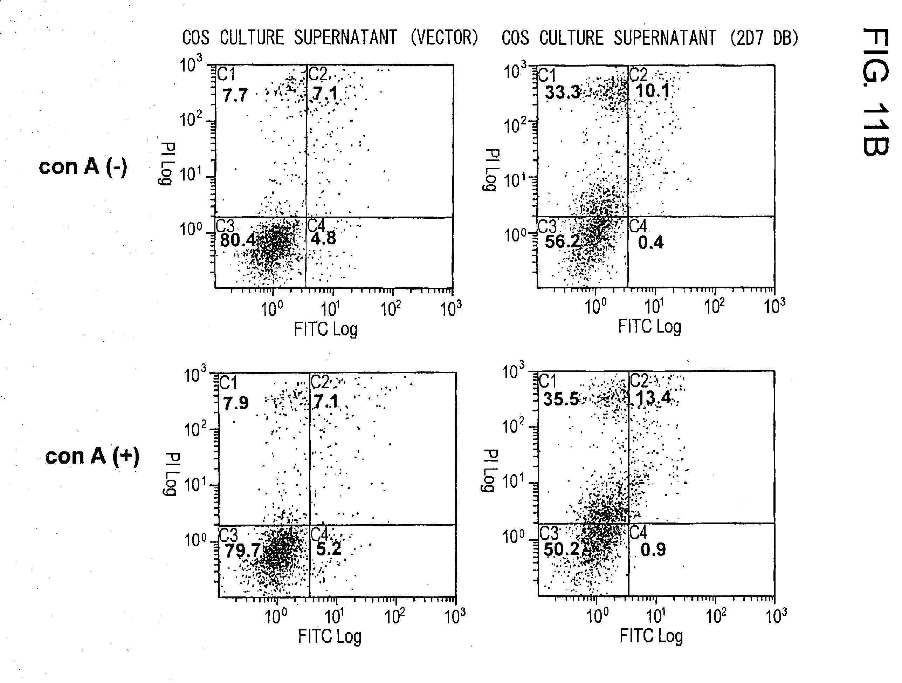

This culture supernatant was added to Jurkat cells at a ratio of 50%. The percentage of dead cells was measured by staining the cells with PI and Annexin V a few days later. No significant change in the apoptosis marker was observed in Jurkat cells to which just the anti-BST-1 antibody and 2D7 antibody (5 µg/ml each) were added. Furthermore, no particular change could be observed when using the culture supernatant of COS7 transfected with the vector alone. On the other hand, cell death was clearly induced in Jurkat cells to which the culture supernatant of COS7 expressing 2D7DB was added (Fig. 11A and Fig. 11B).

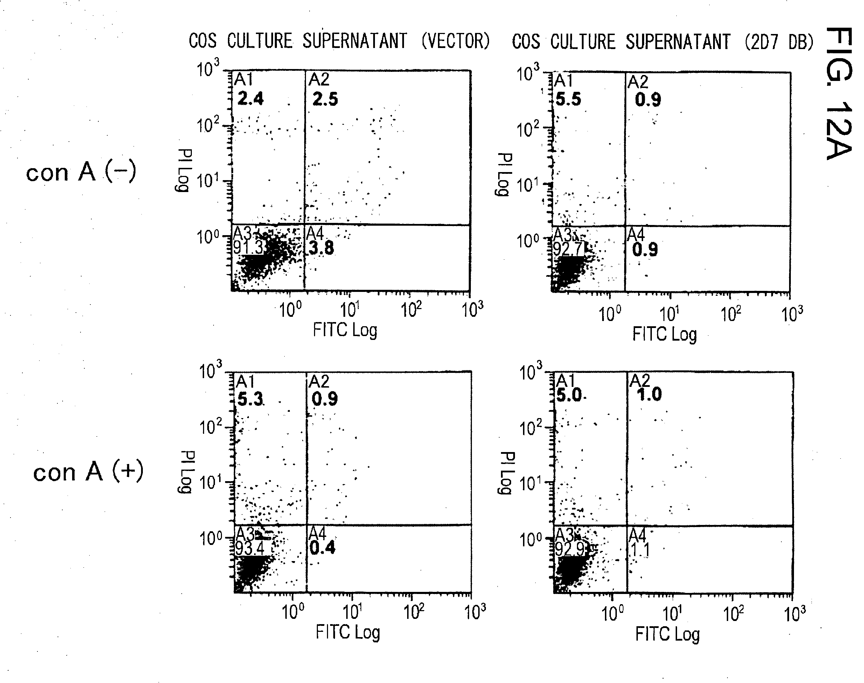

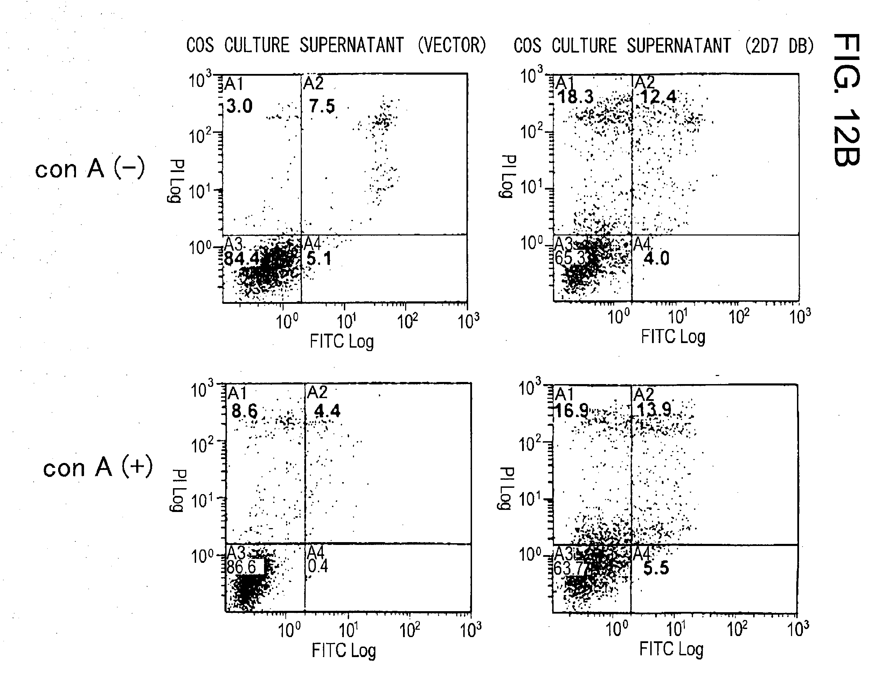

Next, to investigate the HLA class I A-specific action of this 2D7DB, a similar experiment was performed using K562 cells, which are known to not express HLA class I A. As a result, 2D7DB had absolutely no influence on K562 cells, although it showed cell death inducing activity against Jurkat cells (Fig. 12A and Fig. 12B). This strongly supports the idea that the cell death inducing activity of 2D7DB is an action targeting HLA class I A, which is its epitope. Furthermore, according to each data, the sensitivity of Jurkat cells towards 2D7DB was found to be slightly higher in cells stimulated by con A.

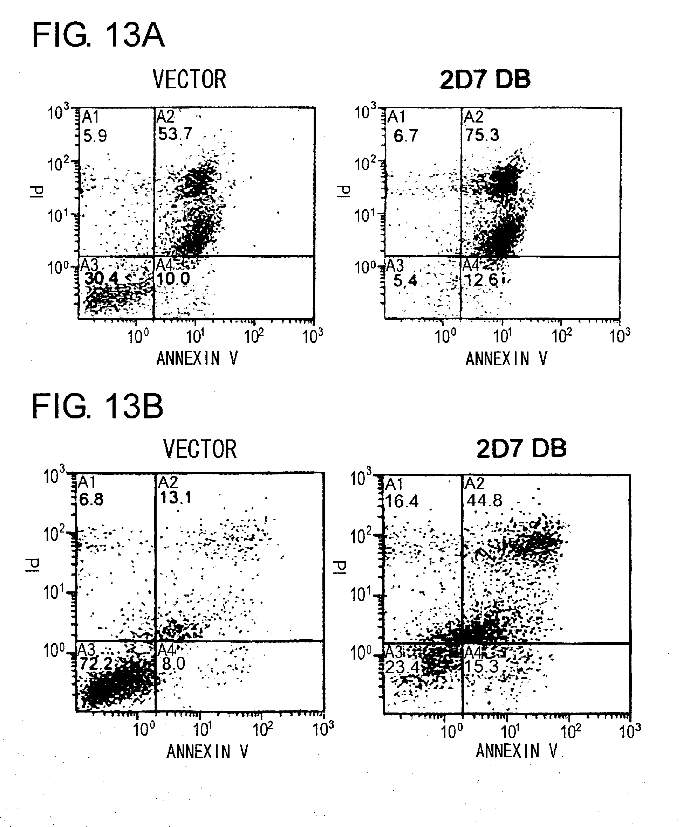

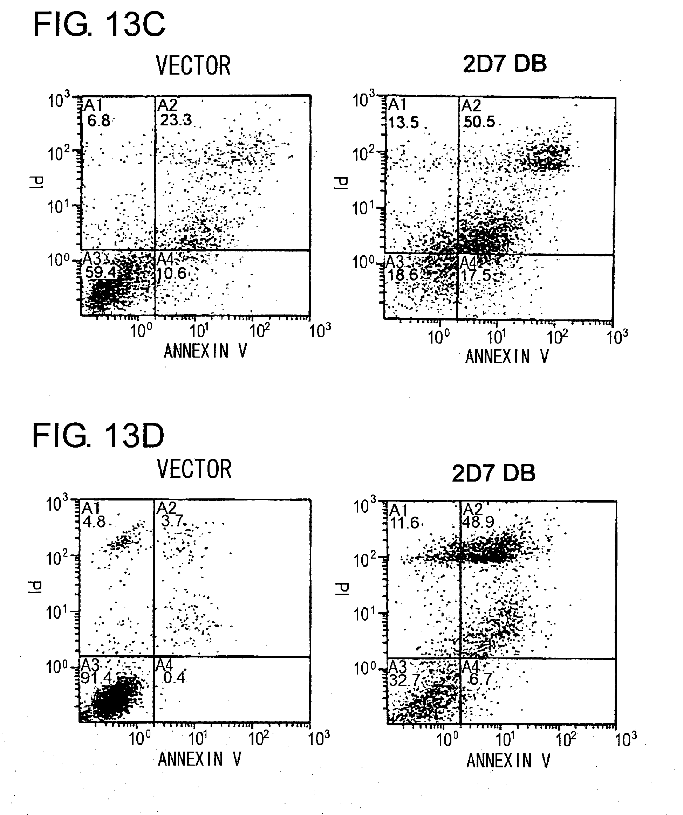

Next, the action of 2D7DB on other myeloma cell lines was analyzed. RPMI8226, IL-KM3, U266, and ARH77 were incubated with a culture supernatant into which the vector alone was transfected (control), or with the 2D7DB-expressing COS7 culture supernatant. Two days later these cultures were double stained with Annexin V and PI, and analyzed using a flow cytometer. As a result, incubation with 2D7DB was found to significantly induce cell death in all of the cells (Fig. 13A to Fig. 13D).

### (ii) Cytotoxic activity of purified 2D7DB

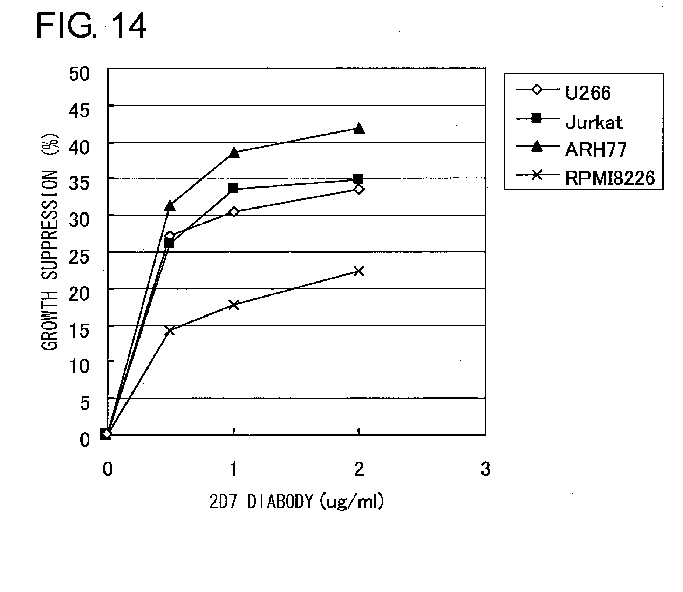

The growth inhibitory effect of purified 2D7DB on each type of cell line (RPMI8226, ARH77, U266, and Jurkat) was analyzed. 2D7DB was added at 0, 0.5, 1.0, and 2.0 µg/ml, and the number of cells was counted three days later. As a result, 2D7DB was found to inhibit cell growth of these cells in a concentration-dependent manner (Fig. 14).

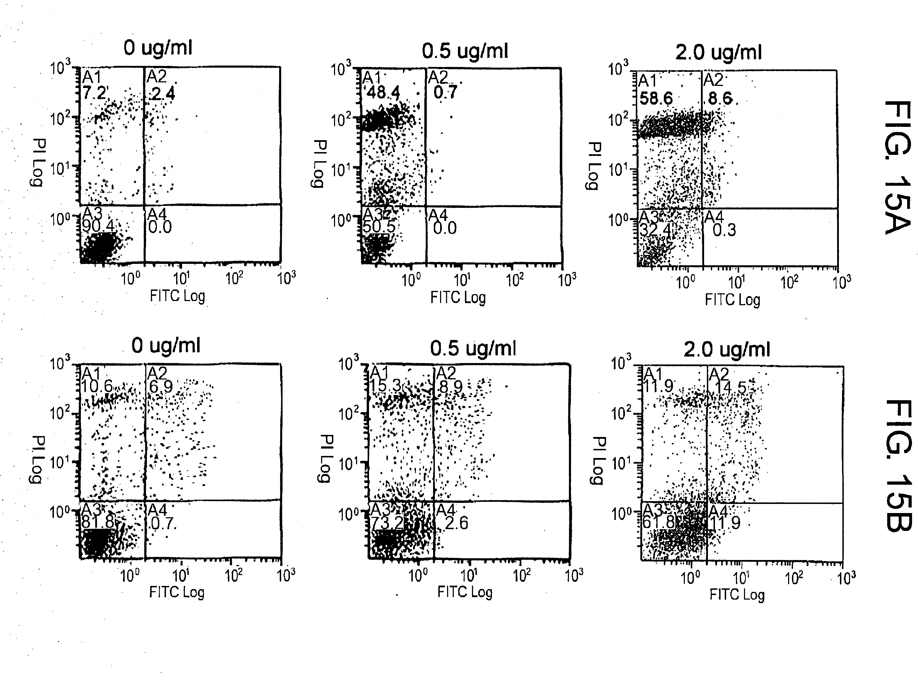

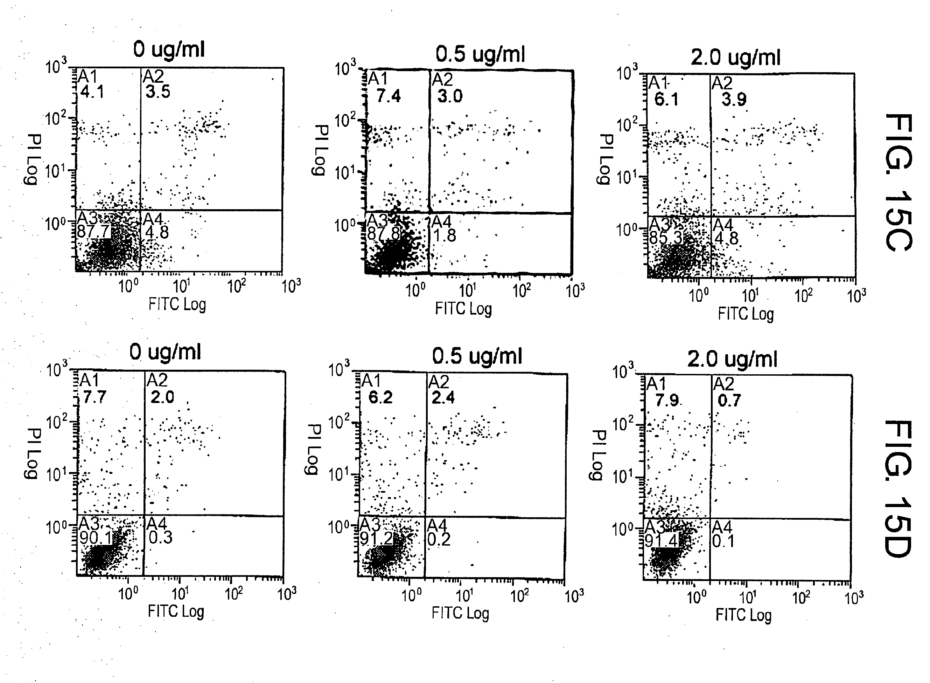

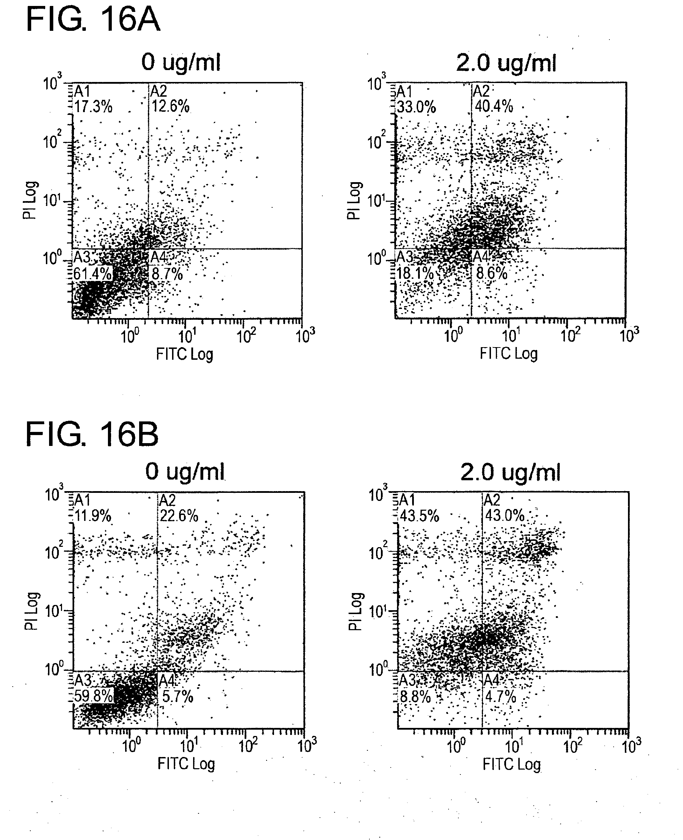

Purified 2D7DB was then added, and 48 hours later, cells were stained with cell death markers, PI and Annexin V, and then analyzed. As in the results obtained when using 2D7DB transiently expressed in COS7, cell death was induced in Jurkat and ARH77 in a concentration-dependent manner, and K562 was not affected at all (Fig. 15A to Fig. 15C). Furthermore, 48 hours after the addition of 2D7DB to U266 and IL-KM3, significant cell death inducing activity was confirmed (Fig. 16A and Fig. 16B).

On the other hand, although the 2D7 antibody stained the adherent HeLa cells very well, 2D7DB had absolutely no influence under the same conditions (Fig. 15 D). This suggested that 2D7DB may act specifically on non-adherent cells, such as blood cells.

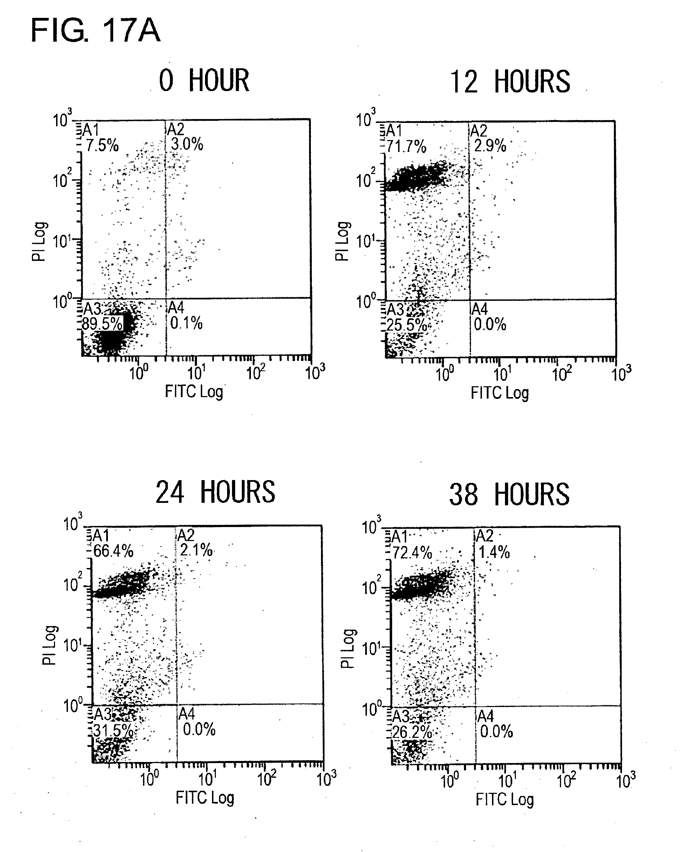

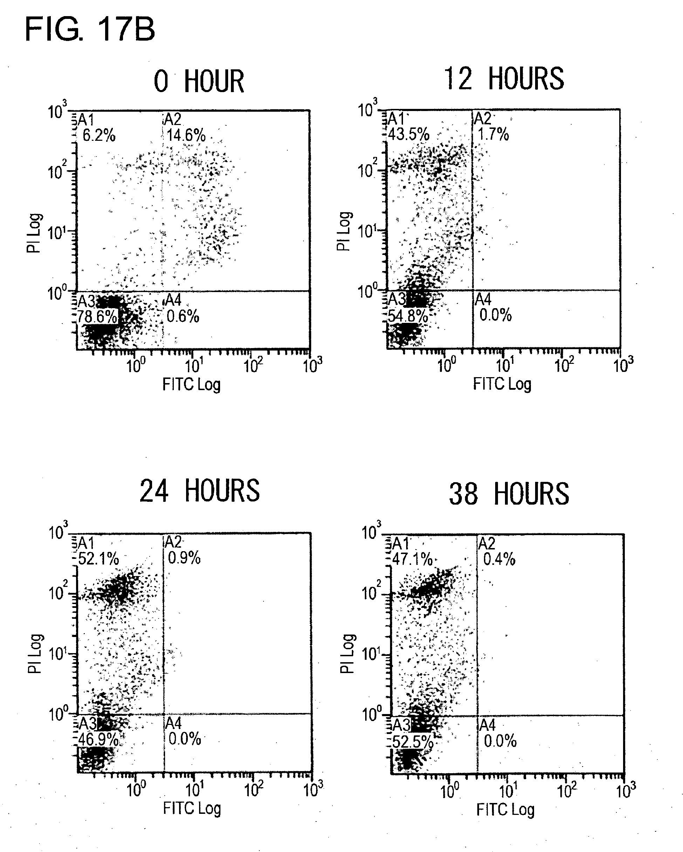

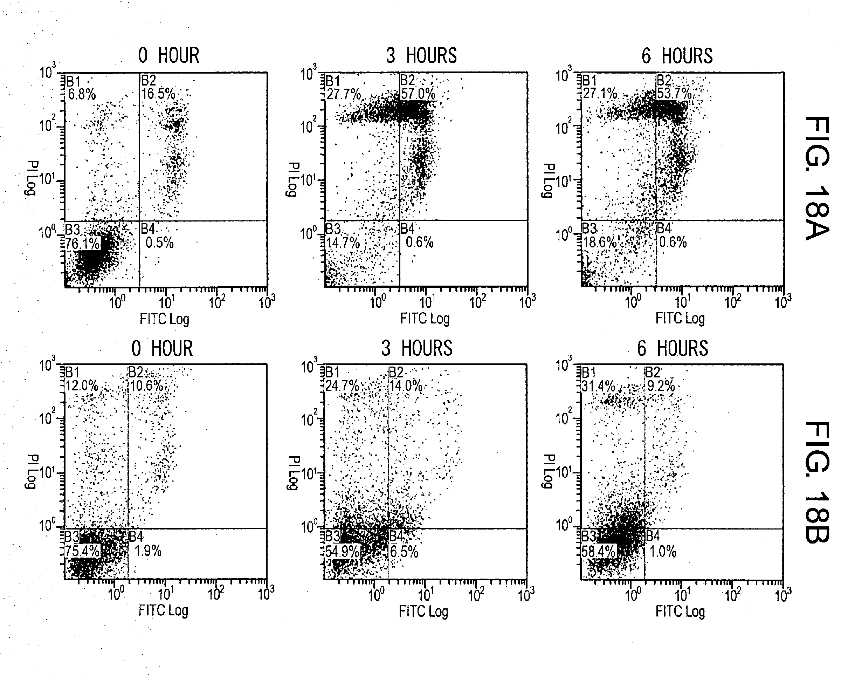

Next, the time taken for 2D7DB to induce cell death was analyzed. 2 µg/ml of 2D7DB was added to ARH77 and Jurkat cells, cells were collected 12, 24, and 38 hours later, and stained with a cell death marker. The results showed that cell death was already induced in all cells twelve hours later (Fig. 17A and Fig. 17B). Therefore, cell death induction was investigated at earlier times (three and six hours). Surprisingly, it was shown that 2D7DB induces cell death at least within three hours after its addition (Fig. 18A and Fig. 18B). These results strongly support the idea that 2D7DB has a very strong cell death-inducing activity. Since 2D7DB strongly induces cell death, sufficient drug efficacy can be expected even with a short half life in the blood. Furthermore, safety becomes a concern if the whole antibody has strong cell death-inducing activity, considering the length of the half life in the blood; however, producing a diabody can overcome such problems.

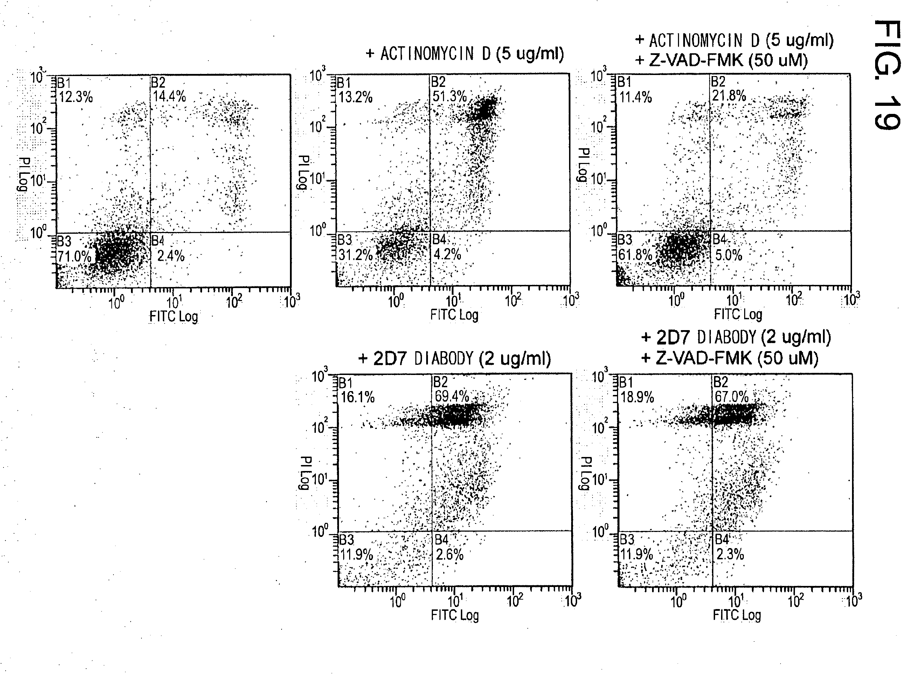

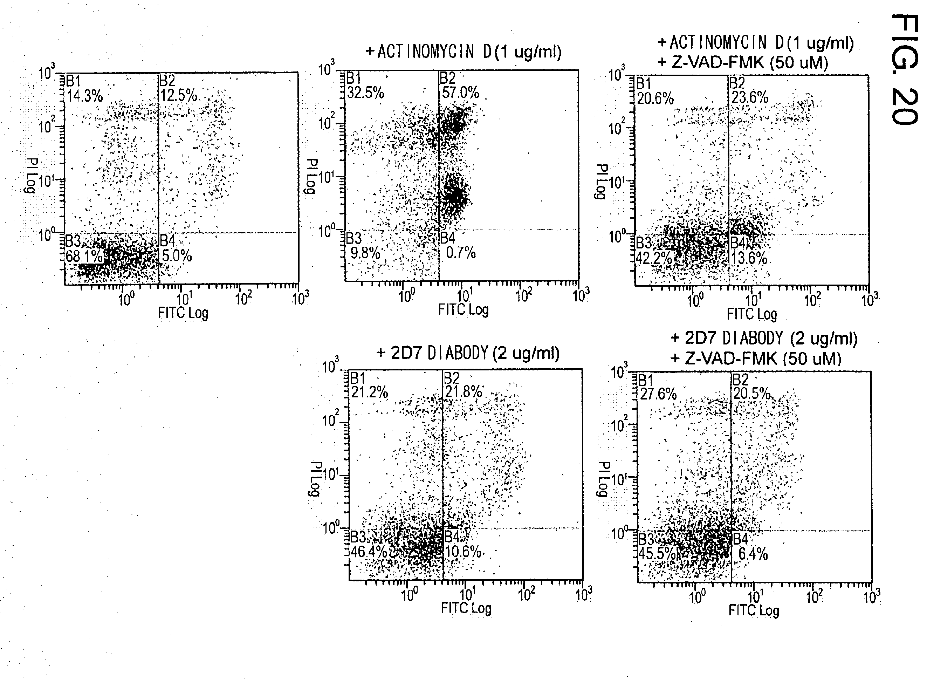

Next, analyses were performed to determine whether cell death due to 2D7DB is induced through caspase activation, that is, whether it is apoptosis. As shown in Fig. 19 and Fig. 20, significant apoptosis was induced when ARH77 and Jurkat cells were treated with the apoptosis-inducing agent Actinomycin D, and then stained 16 hours later with Annexin V and PI. After pre-treating cells under these conditions with caspase inhibitor Z-VAD-FMK for 2.5 hours, apoptosis due to Actinomycin D was suppressed. However, cell death induced by 2D7DB was not inhibited at all by pretreatment with Z-VAD-FMK. These results show that 2D7DB induces cell death by a mechanism different from the ordinary caspase-mediated apoptosis mechanism.

To confirm this, fragmentation of chromatin DNA, known to be the most characteristic biochemical change accompanying apoptosis, was also analyzed.

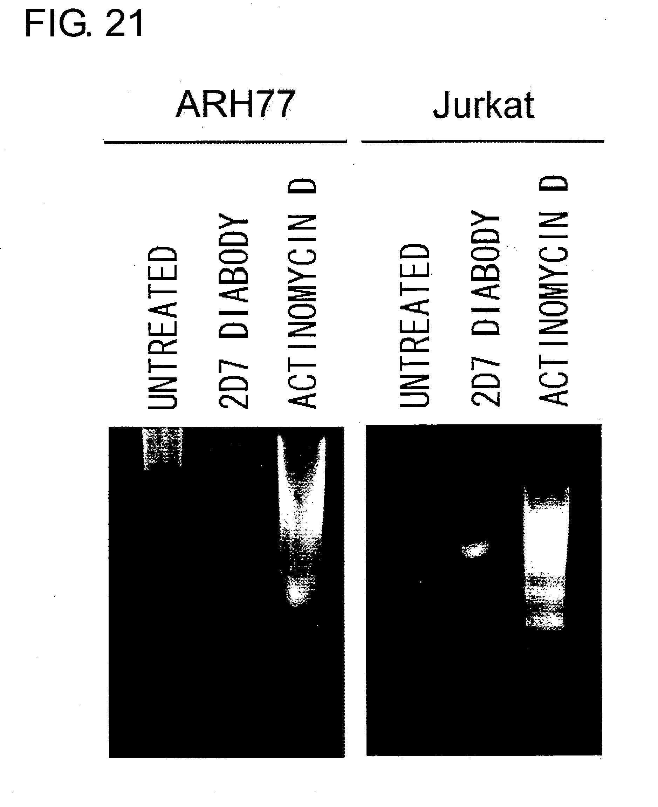

ARH77 and Jurkat cells were treated with 2D7DB (2 µg/ml) or Actinomycin D, and DNAs were collected from the cells 24 hours later and subjected to electrophoresis (Fig. 21). As a result, DNA fragmentation characteristic of apoptosis was induced in all cells treated with Actinomycin D, which is an apoptosis-inducing agent. On the other hand, DNA fragmentation was not observed at all in 2D7DB-treated cells, even though the concentration of added 2D7DB was absolutely sufficient to induce cell death. These results also strongly support the idea that cell death due to 2D7DB is an unknown type of cell death, unaccompanied by the characteristics of apoptosis.

From the above-mentioned results, cell death due to 2D7DB was found to be caused by a pathway different from previously known cell death induction mechanisms. Therefore, further analysis was performed to elucidate the mechanism of cell death induction by 2D7DB. From the experiments described above, when 2D7DB was reacted with the cells, the cell membranes were often observed to be destroyed under the microscope. Therefore, 2D7DB was presumed to have some sort of influence on the actin skeleton. To examine this possibility, the cells were treated with an actin polymerization inhibitor (cytochalasin D), and then the influence of 2D7DB on cell death induction activity was analyzed.

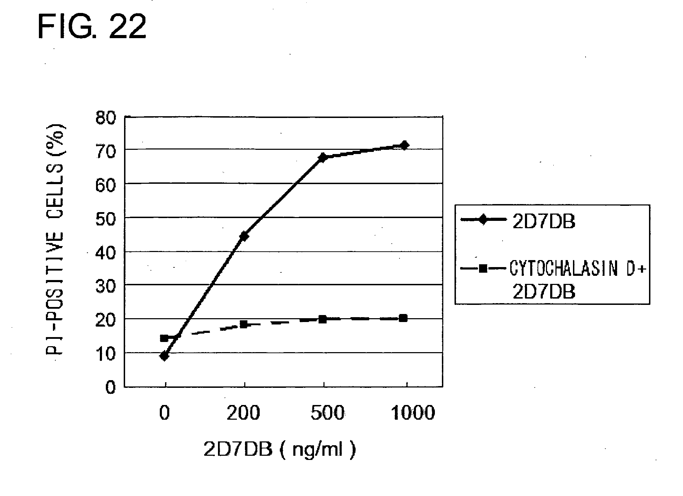

Cytochalasin D (20 µg/ml) or ethanol alone (control) was added to ARH77 cells, and 1 hour later, 2D7DB was added at various concentrations. After a 4-hour incubation from the 2D7DB addition, cells were collected, PI staining was performed and the percentage of dead cells was measured (Fig. 22). As a result, pretreatment of cells with cytochalasin D was found to cause loss of sensitivity towards 2D7DB. These results suggested that 2D7DB causes some kind of effect on the cytoskeletal system such as actin to induce cell death by binding to HLA-class IA, which is the target molecule.

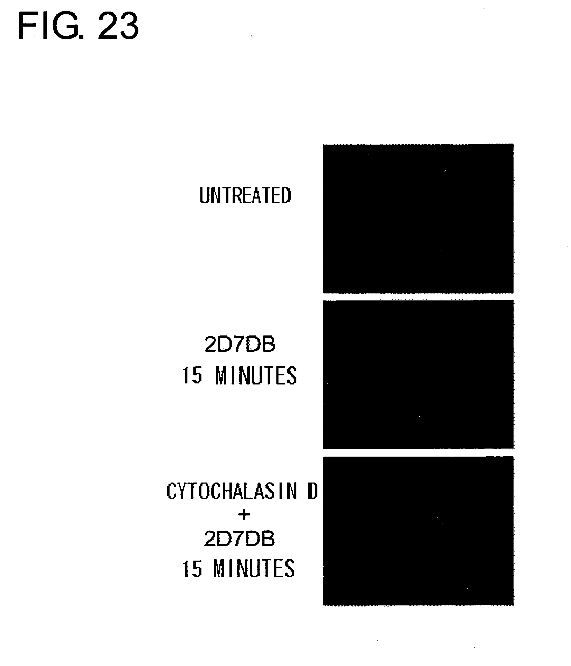

Therefore, cells treated with 2D7DB were stained by the actin antibody, and the dynamic change of the cytoskeletal system due to 2D7DB addition was analyzed visually. ARH77 cells were treated with 2D7DB, and 15 minutes later, the cells were immobilized with methanol, and the state of actin (red) in the cells was investigated by immunostaining (Fig. 23). As a result, compared to the image from those not treated with 2D7DB, significant destruction of the actin skeleton in the cell due to 2D7DB was observed.

The above-mentioned results strongly suggested that cell death due to 2D7DB may be caused by destruction of the actin skeleton in cells by 2D7DB bound to HLA class IA. This is a completely new type of cell death induction mechanism that has not been reported to date.

### [Example 6] Drug efficacy test for 2D7 diabody using human myeloma animal model

### (1) Production of mouse model for human myeloma

A mouse model of human myeloma was produced as follows. ARH 77 cells were prepared to reach 2.5x 10⁷ cells/ml in RPMI1640 medium (GIBCO BRL) supplemented with 10% fetal calf serum (GIBCO BRL), and then 200 µL of the above-mentioned ARH77 cell suspension (5x 10⁶ cells/mouse) was injected to SCID mice (male, 6 weeks old, Clea Japan) pretreated the day before with intraperitoneal administration of 0.2 mg of anti-asialo GM1 antibody (Wako Pure Chemicals) from the tail vein.

### (2) Preparation of the antibody to be administered

On the day of administration, a 2D7 diabody was prepared at 0.8 mg/ml using filter-sterilized PBS(-), and this was used as the administration sample.

### (3) Antibody administration

To the mouse model of human myeloma produced in (1), the administration sample prepared in (2) was administered through the tail vein at 10 ml/kg twice a day for 3 days from the first day after engraftment of ARH77 cells. As a negative control (vehicle), filter-sterilized PBS(-) was administered similarly at 10 ml/kg through the tail vein twice a day for 3 days. The antibody-administered group had 7 animals per group, and the vehicle-administered group had 8 animals per group.

### (4) Human IgG assay of mouse serum

The quantity of human IgG produced by human myeloma cells in the mouse serum was determined by ELISA described below. 100 µL of goat anti-human IgG antibody (BIOSOURCE) diluted to 1 µg/ml with 0.1% bicarbonate buffer (pH9.6) was placed into a 96-well plate (Nunc), this was incubated at 4°C overnight, and the antibody was immobilized. After blocking, mouse serum diluted in a stepwise manner, or as a standard, 100 µL of human IgG (Cappel) was added, and this was incubated at room temperature for 1 hour. After washing, 100 µL of a 5000-fold diluted alkaline phosphatase-labeled anti-human IgG antibody (BIOSOURCE) was added, and this was incubated at room temperature for 1 hour. After washing, substrate solution was added, and after incubation, absorbance at 405 nm was measured using MICROPLATE READER Model 3550 (BioRad), and the concentration of human IgG in mouse serum was calculated from the calibration curve obtained from the absorbance of the standard human IgG sample.

### (5) Evaluation of anti-tumor effect

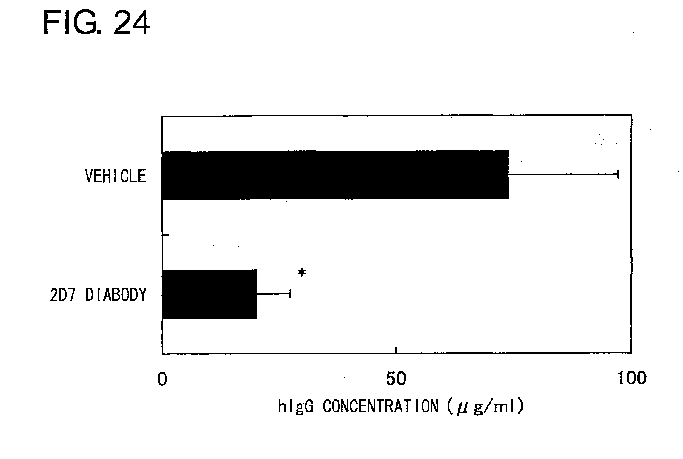

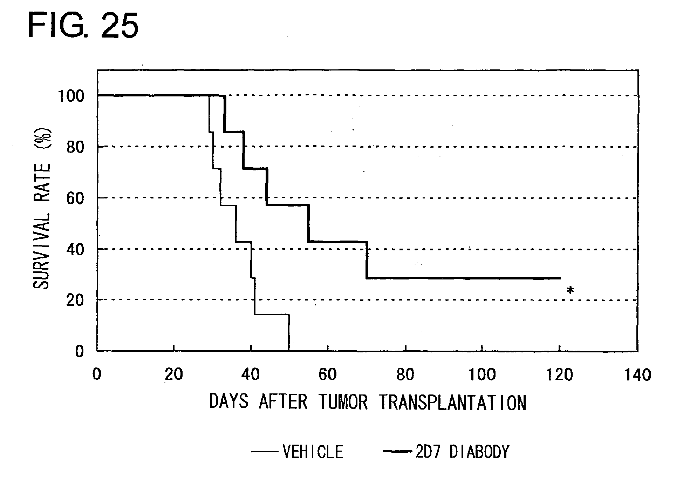

The anti-tumor effect of the 2D7 diabody on a human myeloma mouse model was evaluated using the change in the amount of human IgG (M protein) produced by the myeloma cells in mouse serum, and by the survival time. Regarding the change in human IgG level in mouse serum, serum was collected on the 24th day after transplanting the ARH77 cells, and the human IgG level was measured by the ELISA described above in (4). As a result, the level of human IgG (M protein) in the serum had increased in the vehicle-administered group to approximately 74 µg/ml. In contrast, the level in the 2D7 diabody-administered group was significantly lower than in the control group (P<0.005, unpaired t-test), and 2D7 diabody was shown to very strongly suppress the growth of ARH77 cells (Fig. 24). With regards to survival time, as shown in Fig. 25, the 2D7 diabody-administered group showed a significant increase in survival time compared to the vehicle-administered group.

Accordingly, the 2D7 diabody was shown to have an antitumor effect on the mouse model of human myeloma. The antitumor effect of the 2D7 diabodies of this invention may be based on the cell death-inducing action of this antibody.

### [Example 7] Analysis of the action of 2D7DB on PBMC

The action of 2D7DB on human peripheral blood mononuclear cells (PBMCs) was analyzed. PBMCs were purified from the peripheral blood of a healthy adult volunteer by density gradient centrifugation. The PBMCs were plated at 5x 10⁵ cells/1 ml/well onto a 24-well plate, in the presence or absence of a mitogen. Phytohemagglutinin M (PHA-M, Roche Diagnostics, final concentration: 10 µg/ml), concanavalin A (ConA, Wako, final concentration: 10 µg/ml), and SAC (Pansorbin Cells, Calbiochem, final concentration: 0.01 %) were used as mitogens. Cells were cultured in a 5% CO₂ incubator at 37°C for three days. 24 or 3 hours before culture was complete, 2D7DB was added to yield a final concentration of 2 µg/ml. After culture was complete, the cells were double stained with Annexin V and PI (Annexin V-FITC Apoptosis Detection Kit I, Pharmingen), and then analyzed using a flow cytometer (EPICS XL, Coulter). As a positive control, ARH77 at 2.5x 10⁵ cells/ml/well was cultured for 24 hours in the absence of a mitogen, and was reacted with 2D7DB, as for PBMC.

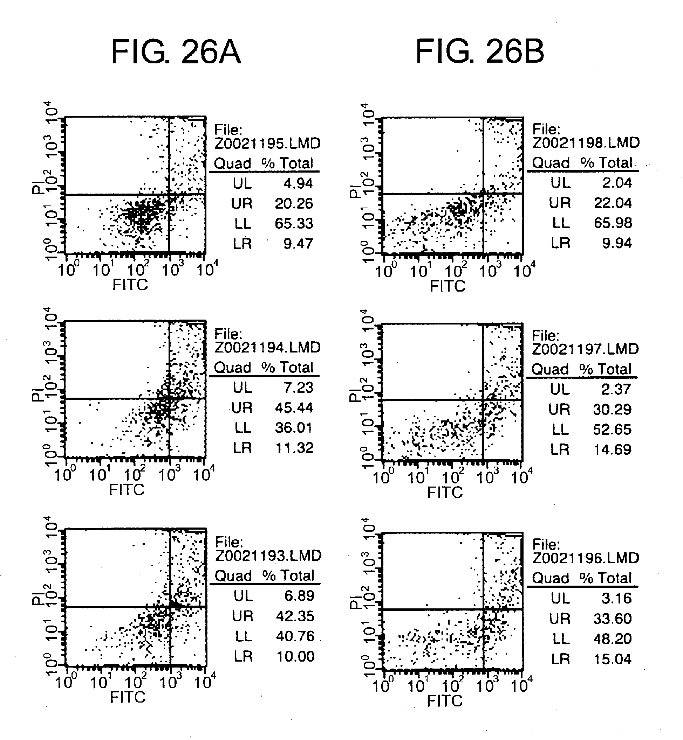

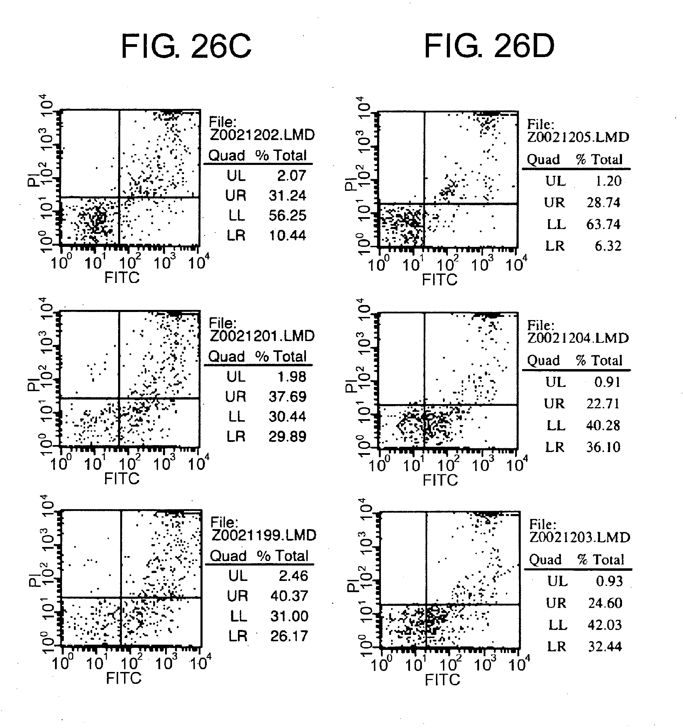

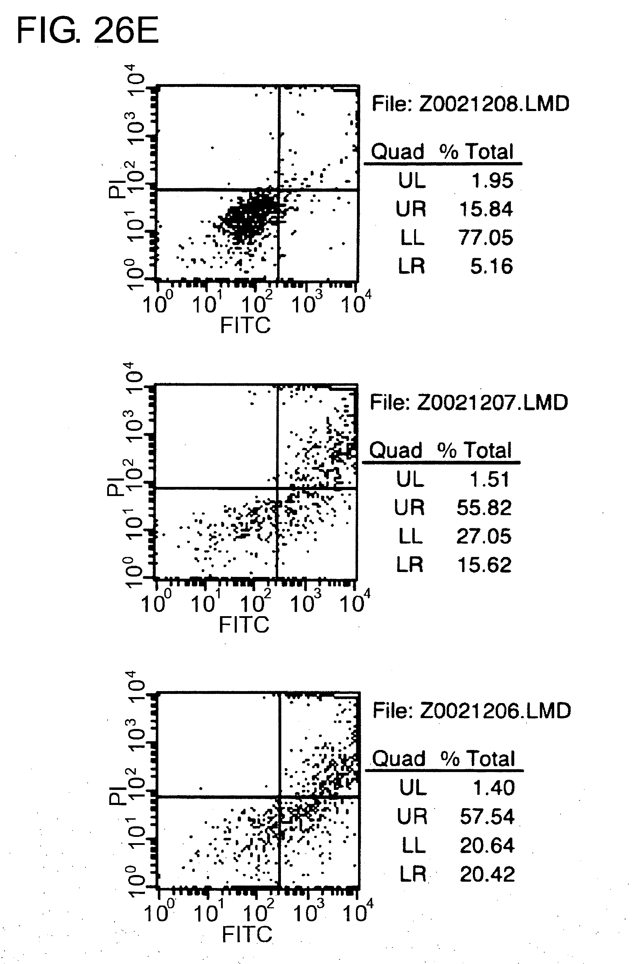

In the case of PBMC, the percentages of dead cells that were both Annexin V and PI-positive were 29%, 23%, and 25% in the absence of mitogens (in order: no addition, 3-hour addition, and 24-hour addition of 2D7DB; continued below); 20%, 45%, and 42% in the presence of PHA-M; 22%, 30%, and 34% in the presence of ConA; and 31%, 38%, and 40% in the presence of SAC (Figs. 26A to 26D). In the case of ARH77, the percentages were 16%, 56%, and 58% (Fig. 26E). These results showed that 2D7DB has hardly any effect on unstimulated PBMC, but induces cell death in a short time with mitogen-activated PBMC.

### Industrial Applicability

This invention provides minibodies with high specific activities. By using these minibodies, adequate drug efficacy can be expected even with a short half-life. The minibodies of the present invention are further expected to be able to improve drug efficacy and to lower toxicity. In addition, since overall cost is reduced, including reduction of clinical dose and production cost, economical problems of concern in the development of antibody pharmaceuticals are also expected to improve.

## Claims

1. A minibody, which comprises a heavy chain variable region comprising CDRs 1, 2, and 3 which consist of the amino acid sequences of SEQ ID NOs: 13, 14, and 15.

2. A minibody which is functionally equivalent to the minibody of claim 1, and which comprises heavy chain CDRs consisting of amino acid sequences with one or more amino acid substitutions, deletions, insertions, and/or additions in the amino acid sequences of the heavy chain CDRs of the minibody of claim 1.

3. A minibody, which comprises a light chain variable region comprising CDRs 1, 2, and 3 which consist of the amino acid sequences of SEQ ID NOs: 16, 17, and 18.

4. A minibody which is functionally equivalent to the minibody of claim 3, and which comprises light chain CDRs consisting of amino acid sequences with one or more amino acid substitutions, deletions, insertions, and/or additions in the amino acid sequences of the light chain CDRs of the minibody of claim 3.

5. A minibody, which comprises a heavy chain variable region comprising CDRs 1, 2, and 3 which consist of the amino acid sequences of SEQ ID NOs: 13, 14, and 15, and a light chain variable region comprising CDRs 1, 2, and 3 which consist of the amino acid sequences of SEQ ID NOs: 16, 17, and 18.

6. A minibody which is functionally equivalent to the minibody of claim 5, and which comprises CDRs consisting of amino acid sequences with one or more amino acid substitutions, deletions, insertions, and/or additions in the amino acid sequences of the CDRs of the minibody of claim 5.

7. The minibody of any one of claims 1 to 6, which is a diabody.

8. A cell death-inducing agent comprising the minibody of any one of claims 1 to 7 as an active ingredient.

9. The cell death-inducing agent of claim 8, which induces cell death of a B-cell or T-cell.

10. The cell death-inducing agent of claim 9, wherein the B-cell or T-cell is an activated B-cell or an activated T-cell.

11. A cell growth-suppressing agent comprising the minibody of any one of claims 1 to 7 as an active ingredient.

12. An anti-tumor agent comprising the minibody of any one of claims 1 to 7 as an active ingredient.

13. The anti-tumor agent of claim 12, wherein the tumor is blood tumor.

14. An agent for treating an autoimmune disease, wherein the agent comprises the minibody of any one of claims 1 to 7 as an active ingredient.


SEQUENCE LISTING

<110> CHUGAI SEIYAKU KABUSHIKI KAISHA
      OZAKI Shuji
      ABE Masahiro

<120> Cell Death Inducer

<130> C1-A0404P

<160> 18

<170> PatentIn version 3.1

<210> 1
<211> 547
<212> DNA
<213> Mus musculus

<400> 1
tacgactcac tatagggcaa gcagtggtat caacgcagag tacgcgggga atctatgatc      60

agtgtcctct ctacacagtc cctgacgaca ctgactccaa ccatgcgatg gagctggatc     120

tttctcttcc tcctgtcaat aactgcaggt gtccattgcc aggtccagtt gcagcagtct     180

ggacctgagc tggtgaagcc tggggcttca gtgaagatgt cttgtaaggc ttctggctac 240

accttcacag actactttat acactgggtg aaacagaggc ctggacaggg acttgaatgg 300

attggatgga tttttcctgg agatgatact actgattaca atgagaagtt caggggcaag 360

accacactga ctgcagacaa atcctccagc acagcctaca ttttgctcag cagcctgacc 420

tctgaggact ctgcgatgta tttctgtgta aggagtgacg actttgacta ctggggccag 480

ggcaccactc tcacagtctc ctcagccaaa acaacacccc catcagtcta tccactggcc 540

cctgctg 547

<210> 2

<211> 535

<212> DNA

<213> Mus musculus

<400> 2

ctaatacgac tcactatagg gcaagcagtg gtatcaacgc agagtacgcg gggactwatg 60

agaatagcag taattagcta gggaccaaaa ttcaaagaca aaatgcattt tcaagtgcag 120

attttcagct tcctgctaat cagtgcctca gtcatcatgt ccagaggaca aattgttctc 180

acccagtcgc cagcaatcat gtctgcatct ccaggggaga aggtcaccat aacctgcagt 240

gccagctcaa gtgtaagtta catgcactgg ttccagcaga agccaggcac ttttcccaaa 300

ctctggattt atagcacatc caacctggct tctggagtcc ctactcgctt cagtggcagt 360

ggatctggga cctcttactc tctcacaatc agccgaatgg aggctgaaga tgctgccact 420

tattactgcc agcaaaggac gagttatcca cccacgttcg gctcggggac aaagttggag 480

ataaaacggg ctgatgctgc accaactgta tccatcttcc caccatccag tgagc 535

<210> 3
<211> 789
<212> DNA
<213> Artificial

<220>
<223> an artificially synthesized DNA sequence

<220>
<221> CDS
<222> (14)..(775)
<223>

<400> 3

cctgaattcc acc atg cga tgg agc tgg atc ttt ctc ttc ctc ctg tca 49

Met Arg Trp Ser Trp Ile Phe Leu Phe Leu Leu Ser

1               5                   10

ata act gca ggt gtc cat tgc cag gtc cag ttg cag cag tct gga cct 97

Ile Thr Ala Gly Val His Cys Gln Val Gln Leu Gln Gln Ser Gly Pro

        15                  20                  25

gag ctg gtg aag cct ggg gct tca gtg aag atg tct tgt aag gct tct 145

Glu Leu Val Lys Pro Gly Ala Ser Val Lys Met Ser Cys Lys Ala Ser

    30                  35                  40

ggc tac acc ttc aca gac tac ttt ata cac tgg gtg aaa cag agg cct 193

Gly Tyr Thr Phe Thr Asp Tyr Phe Ile His Trp Val Lys Gln Arg Pro

45                  50                  55                  60

gga cag gga ctt gaa tgg att gga tgg att ttt cct gga gat gat act 241

Gly Gln Gly Leu Glu Trp Ile Gly Trp Ile Phe Pro Gly Asp Asp Thr

                65                  70                  75

act gat tac aat gag aag ttc agg ggc aag acc aca ctg act gca gac 289

Thr Asp Tyr Asn Glu Lys Phe Arg Gly Lys Thr Thr Leu Thr Ala Asp

            80                  85                  90

aaa tcc tcc agc aca gcc tac att ttg ctc agc agc ctg acc tct gag 337
Lys Ser Ser Ser Thr Ala Tyr Ile Leu Leu Ser Ser Leu Thr Ser Glu
        95                  100                 105

gac tct gcg atg tat ttc tgt gta agg agt gac gac ttt gac tac tgg 385
Asp Ser Ala Met Tyr Phe Cys Val Arg Ser Asp Asp Phe Asp Tyr Trp
    110                 115                 120

ggc cag ggc acc act ctc aca gtc tcc tca ggt gga ggc ggt agc caa 433
Gly Gln Gly Thr Thr Leu Thr Val Ser Ser Gly Gly Gly Gly Ser Gln
125                 130                 135                 140

att gtt ctc acc cag tcg cca gca atc atg tct gca tct cca ggg gag 481
Ile Val Leu Thr Gln Ser Pro Ala Ile Met Ser Ala Ser Pro Gly Glu
                145                 150                 155

aag gtc acc ata acc tgc agt gcc agc tca agt gta agt tac atg cac 529
Lys Val Thr Ile Thr Cys Ser Ala Ser Ser Ser Val Ser Tyr Met His
            160                 165                 170

tgg ttc cag cag aag cca ggc act ttt ccc aaa ctc tgg att tat agc 577
Trp Phe Gln Gln Lys Pro Gly Thr Phe Pro Lys Leu Trp Ile Tyr Ser
        175                 180                 185

aca tcc aac ctg gct tct gga gtc cct act cgc ttc agt ggc agt gga 625
Thr Ser Asn Leu Ala Ser Gly Val Pro Thr Arg Phe Ser Gly Ser Gly

    190                 195                 200

tct ggg acc tct tac tct ctc aca atc agc cga atg gag gct gaa gat 673
Ser Gly Thr Ser Tyr Ser Leu Thr Ile Ser Arg Met Glu Ala Glu Asp
205                 210                 215                 220

gct gcc act tat tac tgc cag caa agg acg agt tat cca ccc acg ttc 721
Ala Ala Thr Tyr Tyr Cys Gln Gln Arg Thr Ser Tyr Pro Pro Thr Phe
                225                 230                 235

ggc tcg ggg aca aag ttg gag ata aaa gac tac aag gat gac gac gat 769
Gly Ser Gly Thr Lys Leu Glu Ile Lys Asp Tyr Lys Asp Asp Asp Asp
            240                 245                 250

aag tga taagcggccg caat 789
Lys

<210> 4
<211> 253
<212> PRT
<213> Artificial

<220>
<223> an artificially synthesized peptide sequence

<400> 4

Met Arg Trp Ser Trp Ile Phe Leu Phe Leu Leu Ser Ile Thr Ala Gly

1               5                   10                  15

Val His Cys Gln Val Gln Leu Gln Gln Ser Gly Pro Glu Leu Val Lys

            20                  25                  30

Pro Gly Ala Ser Val Lys Met Ser Cys Lys Ala Ser Gly Tyr Thr Phe

        35                  40                  45

Thr Asp Tyr Phe Ile His Trp Val Lys Gln Arg Pro Gly Gln Gly Leu

    50                  55                  60

Glu Trp Ile Gly Trp Ile Phe Pro Gly Asp Asp Thr Thr Asp Tyr Asn

65                  70                  75                  80

Glu Lys Phe Arg Gly Lys Thr Thr Leu Thr Ala Asp Lys Ser Ser Ser

                85                  90                  95

Thr Ala Tyr Ile Leu Leu Ser Ser Leu Thr Ser Glu Asp Ser Ala Met

            100                 105                 110

Tyr Phe Cys Val Arg Ser Asp Asp Phe Asp Tyr Trp Gly Gln Gly Thr

        115                 120                 125

Thr Leu Thr Val Ser Ser Gly Gly Gly Gly Ser Gln Ile Val Leu Thr

    130                 135                 140

Gln Ser Pro Ala Ile Met Ser Ala Ser Pro Gly Glu Lys Val Thr Ile
145                 150                 155                 160

Thr Cys Ser Ala Ser Ser Ser Val Ser Tyr Met His Trp Phe Gln Gln
                165                 170                 175

Lys Pro Gly Thr Phe Pro Lys Leu Trp Ile Tyr Ser Thr Ser Asn Leu
            180                 185                 190

Ala Ser Gly Val Pro Thr Arg Phe Ser Gly Ser Gly Ser Gly Thr Ser
        195                 200                 205

Tyr Ser Leu Thr Ile Ser Arg Met Glu Ala Glu Asp Ala Ala Thr Tyr
    210                 215                 220

Tyr Cys Gln Gln Arg Thr Ser Tyr Pro Pro Thr Phe Gly Ser Gly Thr
225                 230                 235                 240

Lys Leu Glu Ile Lys Asp Tyr Lys Asp Asp Asp Asp Lys
                245                 250

<210> 5
<211> 29

<212>  DNA

<213>  Artificial

<220>

<223>  an artificially synthesized adapter sequence

<400>  5

aattcccagc acagtggtag ataagtaag                                          29

<210>  6

<211>  29

<212>  DNA

<213>  Artificial

<220>

<223>  an artificially synthesized adapter sequence

<400>  6

tcgacttact tatctaccac tgtgctggg                                          29

<210>  7

<211>  24

<212>  DNA

<213>  Artificial

<220>

<223>  an artificially synthesized primer sequence

<400>  7
caggggccag tggatagact gatg                                              24

<210>  8
<211>  23
<212>  DNA
<213>  Artificial

<220>

<223>  an artificially synthesized primer sequence

<400>  8
gctcactgga tggtgggaag atg                                               23

<210>  9
<211>  35
<212>  DNA
<213>  Artificial

<220>

<223>  an artificially synthesized primer sequence

<400>  9
cctgaattcc accatgcgat ggagctggat ctttc                                35

<210>  10
<211>  47
<212>  DNA
<213>  Artificial

<220>
<223>  an artificially synthesized primer sequence

<400>  10
aatttggcta ccgcctccac ctgaggagac tgtgagagtg gtgccct                   47

<210>  11
<211>  47
<212>  DNA
<213>  Artificial

<220>
<223>  an artificially synthesized primer sequence

<400>  11

tcctcaggtg gaggcggtag ccaaattgtt ctcacccagt cgccagc                    47

<210>  12

<211>  68

<212>  DNA

<213>  Artificial

<220>

<223>  an artificially synthesized primer sequence

<400>  12

attgcggccg cttatcactt atcgtcgtca tccttgtagt cttttatctc caactttgtc      60

cccgagcc                                                                68

<210>  13

<211>  5

<212>  PRT

<213>  Mus musculus

<400>  13

Asp Tyr Phe Ile His

1               5

<210> 14

<211> 17

<212> PRT

<213> Mus musculus

<400> 14

Trp Ile Phe Pro Gly Asp Asp Thr Thr Asp Tyr Asn Glu Lys Phe Arg

1               5                   10                  15

Gly

<210> 15

<211> 6

<212> PRT

<213> Mus musculus

<400> 15

Ser Asp Asp Phe Asp Tyr

1               5

<210> 16

<211> 10

<212> PRT

<213> Mus musculus

<400> 16

Ser Ala Ser Ser Ser Val Ser Tyr Met His

1               5                   10

<210> 17

<211> 7

<212> PRT

<213> Mus musculus

<400> 17

Ser Thr Ser Asn Leu Ala Ser

1               5

<210> 18

<211> 9

<212> PRT

<213> Mus musculus

<400> 18

Gln Gln Arg Thr Ser Tyr Pro Pro Thr

1               5